# EUROPÄISCHE PATENTSCHRIFT

(11) **EP 0 423 747 B1**
(45) Veröffentlichungstag und Bekanntmachung des Hinweises auf die Patenterteilung: **22.07.1998**
(21) Anmeldenummer: 90119886.1
(22) Anmeldetag: 17.10.1990
(51) Int. Cl.: C07H 17/04, A61K 31/70, A61K 39/395

(54) **Glycosyl-Etoposid-Prodrugs, Verfahren zu ihrer Herstellung und ihre Anwendung in Kombination mit funktionalisierten tumorspezifischen Enzym-Konjugaten**
Glycosyl-etoposide prodrugs, process for their preparation and their use in combination with functionalised tumour-specific enzyme conjugates
Prodrogues glycosyl-étoposide, leur procédé de préparation et leur utilisation en combinaison avec des conjugats enzymatiques conjugués fonctionnalisés spécifiques aux tumeurs

(30) Priorität: 20.10.1989 DE 3935016
(43) Veröffentlichungstag der Anmeldung: 24.04.1991
(73) Patentinhaber: BEHRINGWERKE Aktiengesellschaft, 35001 Marburg (DE)
(72) Erfinder: Kolar, Cenek, Dr., W-3550 Marburg (DE); Czech, Jörg, Dr., W-3550 Marburg (DE); Bosslet, Klaus, Dr., W-3550 Marburg (DE); Seemann, Gerhard, Dr., W-3550 Marburg (DE); Sedlacek, Hans Harald, Dr., W-3550 Marburg (DE)
(74) Vertreter: Fischer, Hans-Jürgen, Dr.

(56) Entgegenhaltungen:
- EP-A- 141 079
- EP-A- 0 302 473
- WO-A-88/07378
- DE-A- 4 002 888
- CANCER RESEARCH, Band 48, Nr. 20, 15. Oktober 1988, Seiten 5692-5695; K. HANDE et al.: "Metabolism and excretion of etoposide in isolated, perfused rat liver models"
- CANCER RESEARCH, Band 48, Nr. 7, 1. April 1988; K. HANDE et al.: "Identification of etoposide glucuronide as a major metabolite of etoposide in the rat and rabbit"
- METHODOLOGICAL SURVEYS IN BIOCHEMISTRY ANAL., Band 16 (Bioactive analytes, incl. CNS drugs, peptides and enantiomers), Seiten 389-393; J.J.M. HOLTHUIS et al.: "Determination of the glucuronide(s) of the antineoplastic agent etoposide"
- Wissenschaftliche Tabellen Geigy Ltd. S. 165-167 ç. Ed 1985
- Methods in Enzymology Band 93, S. 280-333 (1983)
- Nature Band 312, S. 604-608 (1984)
- Cancer Immunology Immunotherapy Band 23, S. 185-191 (1986)
- Eur. J. Nucl. Med. Band 14, S. 523-528 (1988)
- British Journal of Cancer Band 55(2), S. 147-152 (1987)
- Bioorganic & Med. Chem. Lett. Band 4 (21)S. 2567-2572
- Cancer Immuno. Immunother. Band 29, S. 171-178 (1989)

## Beschreibung

Die vorliegende Erfindung bezieht sich auf Glycosyl-Etoposid-Prodrugs, Verfahren zu ihrer Herstellung und ihrer Anwendung in Kombination mit funktionalisierten tumorspezifischen Enzym-Konjugaten für die Behandlung von Krebserkrankungen, und sie betrifft speziell 4'-O-Glycosyl-Etoposide als Prodrugs, die unter Einwirkung von tumorspezifischen Enzym-Konjugaten zu zytotoxischen Wirkstoffen gespalten werden können, wobei der freigesetzte Wirkstoff aufgrund seiner zytostatischen Wirksamkeit zur Behandlung von Krebserkrankungen geeignet ist.

Die Kombination von Prodrug und tumorspezifischen Antikörper-Enzym-Konjugaten zur Anwendung als therapeutische Mittel ist in der Fachliteratur beschrieben. Hierbei wurden gegen bestimmtes Gewebe gerichtete Antikörper, an die ein Prodrug-spaltendes Enzym kovalent gebunden sind, einem Tier, welches das transplantierte Gewebe enthält, injiziert, und anschließend wird eine Enzym-aktivierbare Prodrug-Verbindung verabreicht. Unter der Einwirkung des am Gewebe verankerten Antikörper-Enzym-Konjugates wird das Prodrug zum Zellgift umgewandelt, das eine zytotoxische Wirkung gegen das transplantierte Gewebe ausübt.

In WO 88/07378 ist ein zwei Komponenten enthaltendes therapeutisches System, bestehend aus einer Antikörper-Enzym-Komponente und einer Enzym-aktivierbaren Prodrug-Komponente, beschrieben. Zur Herstellung der Antikörper-Enzym-Konjugate wird hierbei die Verwendung von Nicht-Säuger-Enzymen beschrieben und die von endogenen Enzymen aufgrund der unspezifischen Wirkstoff-Freisetzung ausgeschlossen. Da die exogenen Enzyme von dem Organismus als Fremdantigene erkannt werden, ist deren Verwendung mit dem Nachteil einer Immunantwort gegenüber diesen nicht körpereigenen Stoffen verbunden, aufgrund deren das am Antikörper immobilisierte Enzym desaktiviert und gegebenenfalls das ganze Konjugat eliminiert wird. Weiterhin werden hier p-Bis-N-(2- chlorethyl)aminobenzylglutaminsäure und deren Derivate als Prodrug verwendet, deren chemische Halbwertzeit nur 5,3 bis 16,5 Stunden beträgt. Die chemische Instabilität eines Prodrugs ist aufgrund der zu erwartenden Nebenwirkungen von Nachteil.

In EPA 0302473 A2 wird ebenfalls ein zwei Komponenten enthaltendes therapeutisches System beschrieben, in dem das am Tumorgewebe lokalisierte Antikörper-Enzym-Konjugat eine Prodrug-Verbindung zu einem zytotoxischen Wirkstoff spaltet. Die hierbei unter anderem beschriebene kombinierte Verwendung von Etoposid-4'-phosphat und deren Derivaten als Prodrug und antikörper-immobilisierten alkalischen Phosphatasen zur Freisetzung der Etoposide ist aufgrund der starken Präsenz von endogenen alkalischen Phosphatasen im Serum von Nachteil. Wie in DE 3826562 A1 beschrieben, werden bereits die Etoposid-4'-phosphate alleinig als therapeutisches Antitumormittel angewandt, wobei die im Serum präsenten Phosphatasen das Etoposid aus dem Prodrug freisetzen.

Es zeigte sich überraschenderweise, daß die synthetisch hergestellte, bisher unzugängliche Verbindung 4'-0- alpha-D-glucopyranosyl-etoposid mit dem Enzym alphaglucosidase sowie einem tumorspezifischen Antikörper-Glucosidase-Konjugat in vitro in Etoposid und D-Glucose aufspaltbar ist.

Ausgehend von dieser Erkenntnis, sowie unter Berücksichtigung der oben beschriebenen Nachteile von Kombinationen von Prodrugs und Antikörper-Enzym-Konjugaten hatte es sich die vorliegende Erfindung zur Aufgabe gestellt, synthetische, enzymatisch spaltbare 4'-0-Glycosyl-Etoposide sowie funktionalisierte tumorspezifische Enzyme bereitzustellen und die pharmakologische Nützlichkeit der Kombination der beiden Komponenten in geeigneten Säuger-Testmodellen zu prüfen. Gelöst wurde diese Aufgabe durch die Bereitstellung von Verbindungen der Formel I sowie von funktionalisierten tumorspezifischen Enzymen, die bei ihrer kombinierten Anwendung in der Prüfung auf zytostatische Wirksamkeit eine Wirkung zeigten.

Gegenstand der Erfindung sind 4'-O-Glycosyl-Etoposide der allgemeinen Formel I, 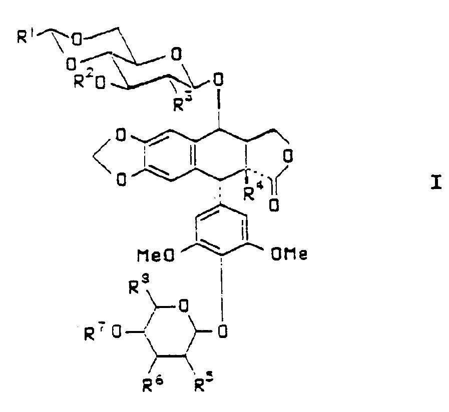 worin
R¹ eine Methyl-, Benzyl- oder 2-Thienylgruppe,
R² ein Wasserstoffatom, eine Acyl- oder Tri-C₁-C₄-alkylsilyl-Schutzgruppe,
R³ eine Hydroxygruppe, eine über ein Sauerstoffatom gebundene Acyl- oder Tri-C₁-C₄-alkylsilyl-Schutzgruppe, eine Amino-, Acetylamino-Benzyloxycarbonylamino- oder Dimethylaminogruppe,
R⁴ ein Wasserstoffatom oder eine Methylgruppe,
R⁵ ein Wasserstoffatom, eine Hydroxygruppe, eine über ein Sauerstoffatom gebundene Acyl- oder Tri-Cₗ-C₄- alkylsilyl-Schutzgruppe, eine Amino-, Benzyloxycarbonylamino-, Azido- oder Acetylaminogruppe,
R⁶ eine Hydroxygruppe, eine über ein Sauerstoffatom gebundene Acyl-, oder Tri-C₁-C₄-alkylsilyl-Schutzgruppe, eine Amino-, Benzyloxycarbonylamino- oder Azidogruppe,
R⁷ ein Wasserstoffatom, eine Acyl- oder Tri-C₁-C₄-alkylsilyl- Schutzgruppe und
R⁸ eine Methyl-, Hydroxymethyl- oder eine über eine Methylenoxy-Gruppe gebundene Acyl-Schutzgruppe oder eine Benzyloxycarbonylgruppe,

wobei unter einer Acylschutzgruppe eine Acetyl-, Mono-, Di- oder Trihalogenacetylgruppe mit Halogen, Fluor oder Chlor verstanden werden soll, darstellen.

Unter einem funktionalisierten tumorspezifischen Enzym soll im Rahmen der Erfindung ein Enzym der Formel II 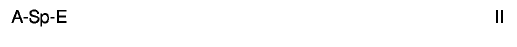 verstanden werden, worin,
A einen Antikörper oder eines seiner Fragmente, die Spezifität zu einem tumor-assoziierten Antigen aufweisen, oder ein in einem Tumor sich anreicherndes Biomolekül, wie EGF (Epidermal Growth Factor), TGF-alpha (Transforming Growth Factor alpha), PDGF (Platelet derived Growth Factor), IGF I+II (Insulin like Growth Factor I+II) oder a+b FGF (acidic + basic Fibroblast Growth Factor)
E eine nicht immunogene oder wenig immunogene Glycosidase, bevorzugt Säugetierglycosidase, wie alpha- oder beta-Glucosidase, alpha-Galactosidase, alpha- oder beta-Mannosidase, alpha-Fucosidase, N-Acetyl-alpha-galactosaminidase, N-Acetyl-beta-/N-Acetyl-alpha-glucosaminidase oder beta-Glucuronidase,
Sp (Spacer) eine bifunktionelle Sulfid- oder Disulfid enthaltende Gruppe der Formel III oder IV 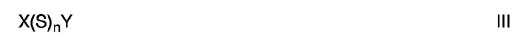 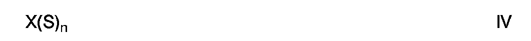 oder einen Polypeptid-Spacer darstellt, worin
X oder Y -CO-R⁹⁻(N-succinimido)- oder -C (=R¹⁰)-CH₂-CH₂- mit R⁹ -CH₂-CH₂-, 1,4-Cyclohexyliden, 1,3 oder 1,4-Phenylen oder Methoxycarbonyl- oder Chloro-phen-1,4-ylen und R¹⁰ O oder NH, und weiterhin
Y -C(=R¹⁰)-CH₂-CH₂-_{'} wobei R¹⁰ die angegebene Bedeutung hat, und
n 1 oder 2

bedeuten.

Das Fusionsgen aus V_{H}, CH1 Hinge und Enzymgen wird in ein Expressionsplasmid kloniert, das für die Expression in eukaryontischen Zellen geeignet ist und einen Selektionsmarker trägt. Das Expressionsplasmid mit dem Fusionsgen wird zusammen mit einem Expressionsplasmid, das das zum Antikörper gehörende leichte Ketten Gen enthält, in eukaryontische Expressionszellen transfiziert. Nach Selektion mit einem geeigneten Antibiotikum werden Transfektomaklone identifiziert, die die Expressionsplasmide enthalten. Durch geeignete Nachweismethoden (BioDot, ELISA) werden solche Transfektomaklone identifiziert, die das Fusionsprotein der Formel II bestehend aus Antikörper und Enzym sezernieren.

Bevorzugt werden im Rahmen der Erfindung Verbindungen der allgemeinen Formel I, worin die Reste
R¹ eine Methyl-, Benzyl- oder 2-Thienylgruppe,
R² ein Wasserstoffatom, eine Acetyl-, Chloroacetyl- oder eine Tri-C₁-C₄-alkylsilyl-Schutzgruppe,
R³ eine Hydroxygruppe, eine über ein Sauerstoffatom gebundene Acetyl-, Chloroacetyl- oder Tri-C₁-C₄-alkyl silyl-Schutzgruppe, eine Amino-, Acetylamino-, Benzyloxycarbonylamino- oder Dimethylaminogruppe,
R⁴ ein Wasserstoffatom oder eine Methylgruppe,
R⁵ ein Wasserstoffatom, eine Hydroxygruppe oder eine über ein Sauerstoffatom gebundene Acetyl-, Chloroacetyl-oder Tri-C₁-C₄-alkylsilyl-Schutzgruppe, eine Amino-, Benzyloxycarbonylamino-, Azido- oder Acetylaminogruppe,
R⁶ eine Hydroxygruppe, eine über ein Sauerstoffatom gebundene Acetyl-, Chloroacetyl- oder Tri-Cᵢ-C₄-alkylsilyl-Schutzgruppe, eine Amino-, Benzyloxycarbonylamino- oder Azidogruppe,
R⁷ ein Wasserstoffatom, eine Acetyl-, Chloroacetyl- oder Tri-C₁-C₄-alkylsilyl-Schutzgruppe und
R⁸ eine Methyl-, Hydroxymethyl-, Acetyloxy- oder Chloroacetyloxymethylgruppe oder eine Benzyloxycarbonylgruppe

darstellen sowie ein funktionalisiertes tumorspezifisches Enzym der Formel II, worin
A ein Antikörper oder dessen Fragment, die Spezifität zu einem Tumor-assoziierten Antigen aufweisen, oder ein am oder im Tumor sich anreicherndes Biomolekül, wie EGF (Epidermal Growth Factor), TGF-alpha (Transforming Growth Factor alpha), PDGF (Platelet derived Growth Factor), IGF I+II (Insulin like Growth Factor I+II), a+b FGF (acidic + basic Fibroblast Growth Factor)
E eine nicht immunogene oder wenig immunogene Glycosidase, bevorzugt eine Saugetierglycosidase, beispielsweise eine alpha- oder beta-Glucosidase, alpha-Galactosidase, alpha- oder beta-Mannosidase, alpha-Fucosidase, N-Acetyl-alpha-galactosaminidase, N-Acetyl-beta-/N-Acetyl-alpha-glucosaminidase oder beta-Glucuronidase,
Sp eine bifunktionellen Disulfid enthaltende Gruppe der Formel II oder IV oder einen Polypeptid-Spacer

darstellen, worin
X oder Y -CO-R⁹⁻(N-succinimido)- oder -C(=R¹⁰)-CH₂-CH₂-mit R⁹ -CH₂-CH₂- oder 1,4-Phenylen und R¹⁰ O oder NH,
Y -C(=R¹⁰)-CH₂-CH₂-, wobei die angegebene Bedeutung hat, und
n 1 oder 2

bedeuten.

Das erfindungsgemäße Verfahren zur Herstellung einer Glycosidase abbaubaren Verbindung der Formel I, worin
R¹ eine Methyl-, Benzyl- oder 2-Thienylgruppe,
R² ein Wasserstoffatom,
R³ eine Hydroxy-, Amino- oder Dimethylaminogruppe,
R⁴ ein Wasserstoffatom oder eine Methylgruppe,
R⁵ ein Wasserstoffatom, eine Hydroxygruppe, eine Amino-oder Acetylaminogruppe,
R⁶ eine Hydroxygruppe oder eine Aminogruppe,
R⁷ ein Wasserstoffatom,
R⁸ eine Methyl- oder Hydroxymethylgruppe oder eine Carboxygruppe oder eine über eine Methylenoxy-Gruppe gebundene Acyl-Schutzgruppe oder eine Benzyloxycarbonylgruppe, wobei unter einer Acylschutzgruppe eine Acetyl-, Mono-, Di- oder Trihalogenacetylgruppe mit Halogen, Fluor oder Chlor verstanden werden soll, sind,

ist dadurch gekennzeichnet, daß man eine Etoposid-Verbindung der Formel V, 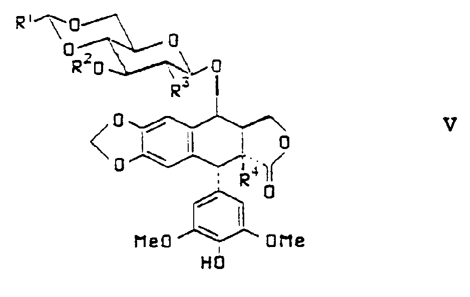 worin
R¹ eine Methyl-, Benzyl- oder 2-Thienylgruppe,
R² ein Wasserstoffatom, eine Acyl-, oder eine Tri-Cᵢ-C₄-alkylsilyl-Schutzgruppe,
R³ eine Hydroxygruppe, eine über Sauerstoff gebundene Acyl- oder Tri-C₁-C₄-alkylsilyl-Schutzgruppe, Acetylamino-, Benzyloxycarbonylamino- oder Dimethylaminogruppe und
R⁴ ein Wasserstoffatom oder eine Methylgruppe darstellen,

mit einer Kohlenhydrat-Komponente der Formel VI, 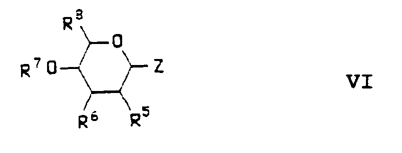 worin
R⁵ ein Wasserstoffatom, eine Hydroxygruppe, eine über ein Sauerstoffatom gebundene Acyl-Schutzgruppe, oder Benzyloxycarbonylamino-, Azido- oder Acetylaminogruppe,
R⁶ eine über ein Sauerstoffatom gebundene Acyl-Schutzgruppe, eine Benzyloxycarbonylamino- oder Azidogruppe, _{R}⁷ eine Acyl-Schutzgruppe,
R⁸ eine Methylgruppe, Methylenoxy-Acyl-Schutzgruppe oder eine Benzyloxycarbonylgruppe und
Z ein Halogenatom, bevorzugt Fluor, Chlor oder Brom, eine Hydroxygruppe, eine Tri-C₁-C₄-alkylsiloxy-Gruppe oder eine über Sauerstoffatom-gebundene Acyl-Schutzgruppe,

wobei die Acyl-Schutzgruppe eine Acetyl-, Mono-, Di- - oder Trihalogen-acetylgruppe, bevorzugt mit Halogenatom Fluor oder Chlor ist, darstellen, in der Gegenwart eines Promotors und gegebenenfalls eines Saurefängers oder Trokkenmittels in einem Losungsmittel bei -50° C bis 60° C zu einem 4'-O-Glycosyl-etoposid-Derivat der Formel I, worin alle Reste R¹ bis R⁸ ihre Bedeutung, wie oben definiert, beibehalten, umsetzt, und in diesen Verbindungen die vorhandenen Schutzgruppen hydrogenolytisch oder hydrolytisch abspaltet und gegebenenfalls eine von den entstandenen Aminogruppen-enthaltenden Verbindungen mittels reduktiver Alkylierung in eine weitere Dimethylaminogruppeenthaltende Verbindung der Formel umsetzt.

Im einzelnen geht man dabei wie folgt vor:
Zur Glycosidierung von Etoposid-Derivaten der Formel V werden funktionalisierte Kohlenhydrat-Bausteine der Formel VI eingesetzt, die typischerweise mit Acyl-Schutzgruppen am 0-2, 0-3, O-4 und gegebenenfalls O-6 Atomen geschützt sind. Bevorzugte Acyl-Schutzgruppen sind Acetyl-, Chloroacetyl- oder Trifluoroacetyl-Gruppen. Bei den Aminozuckern wird die Aminogruppe temporär mit einer Benzyloxycarbonylgruppe oder permanent mit einer Acetylgruppe geschützt. Der Einsatz von Azidozuckern ist ebenfalls möglich, da sie unproblematisch mittels Hydrogenolyse in Aminozucker überführt werden können. Die Kohlenhydrat-Komponenten müssen am anomeren Zentrum geeignet funktionalisiert werden. Hierbei werden Glycosylhalogenide wie Fluoride, Chloride oder Bromide verwendet, die ausgehend von 1-0-Acyl-Derivaten beispielsweise mittels HF, HCI, HBr oder TiBr₄ hergestellt werden können. Die Synthese der Glycosidierungskomponenten, die eine O-Acyl-Gruppe oder eine Hydroxy-Gruppe am anomeren Zentrum tragen, werden nach den in der Kohlenhydratchemie üblichen Verfahren hergestellt.

Die Glycosidierung von Etoposiden der Formel V mit Kohlenhydrat-Bausteinen der Formel VI wird in der Gegenwart eines Promotors durchgeführt. Bei dem Einsatz von Glycosylfluoriden sowie deren 1-Hydroxy- oder 1-Acyloxy-Analoga wird als Promotor BF₃ x Ether oder Trifluormethansulfonsäure tri-Cₗ-C₄-alkylsilylester eingesetzt. Bei Glycosylchloriden oder -bromiden werden als Promotoren Silber- oder Quecksilbersalze eingesetzt.

Die Glycosidierung wird in einem aprotischen organischen Losungsmittel wie Aceton, Ethylacetat, Ether, Toluol, Dichlormethan oder Dichlorethan oder deren Mischungen durchgeführt. Um die bei der Reaktion entstehende Saure oder Wasser abzufangen, werden gegebenenfalls Säurefänger oder Trockenmittel wie Molekularsieb, Magnesiumsulfat zugesetzt. Die Reaktionstemperatur liegt bei dem Einsatz von Glycosylfluoriden und den 1-Hydroxy-Analoga in dem Bereich von -50° C bis 0° C und bei dem Einsatz von Glycosylchloriden oder -bromiden bei 0° C bis 60° C. Die in der Reaktion entstandenen Glycosyletoposide werden nach folgenden Verfahren entblockiert: die Acylschutz-Gruppen werden mittels Zink(11)salz katalysierter Methanolyse oder mit alkalischem Ionenaustauscher in Methanol, Ethanol oder deren Mischung mit Chloroform, Dichloromethan oder Ether entfernt. Die Benzyl- oder Benzyloxycarbonyl-Gruppen oder Azido-Gruppen werden hydrogenolytisch mit Palladium auf Kohle oder Palladium/Bariumsulfat abgespalten oder im Falle der Azidogruppe in Aminogruppe überführt. Die Aminozuckerenthaltenden Verbindungen der Formel 1 können weiterhin zu Dimethylamino-Derivaten mittels reduktiver Alkylierung mit Formaldehyd/Natriumcyanoborhydrid überführt werden.

Zur Herstellung von A-Sp-E kann entweder der Spacer (Sp) über eine Aminogruppe an ein Enzym und an den Antikörper oder das Biomolekül über eine eingeführte oder durch Spaltung der Disulfidbrucke erzeugte HS-Gruppe verknüpft werden, oder es werden Nukleinsäuresequenzen, die für die Teile A, Sp und E kodieren mit Hilfe molekularbiologischer Methoden dergestalt kovalent verknüpft, daß ein Fusionsgen entsteht, und A-Sp-E mit gentechnischen Verfahren hergestellt.

Dies kann auf verschiedene Weisen erfolgen:
A) Eine Restriktionsschnittstelle A wird am 3' Ende des C_{H}1 Exons im Gen der schweren Kette des Immunglobulins mit Hilfe spezifischer Mutagenese eingeführt. Die gleiche Restriktionsschnittstelle A wird am 5' Ende des Oligonukleotids generiert, welches für das Oligopeptid kodiert, das als Spacer fungiert. Beide Restriktionsschnittstellen A werden so gesetzt, daß das Immunglobulingen mit dem Oligonukleotid über die Restriktionsschnittstelle A verknüpft werden kann, ohne das Leseraster zu stören.

Am 3' Ende des Oligonukleotids wird eine Restriktionsschnittstelle B generiert. Diese Restriktionsschnittstelle B wird in dem Gen, das für das Enzym kodiert, an der Stelle eingeführt, an der die kodierende Nukleinsäuresequenz für das reife Protein beginnt. Dann wird das Enzymgen über die Restriktionsschnittstelle B mit dem Immunglobulingen-Linkerkonstrukt verknüpft. Die Restriktionsschnittstellen B werden so gesetzt, daß das Leseraster bei der Verknupfung nicht gestört wird. Das Fusionsgen aus dem für die schwerden Ketten des Immunglobulins V_{H} und C_{H}-1-Linker-Enzym wird in ein Expressionsplasmid kloniert, das für die Expression in eukaryontischen Zellen geeignet ist und einen Selektionsmarker trägt.

Das Expressionsplasmid mit dem Fusionsgen wird zusammen mit einem Expressionsplasmid, das das Gen für die zum Antikörper gehörende leichte Kette trägt, in eukaryontische Zellen transfiziert (z. B. Myelomzellen). Durch Selektion mit geeigneten Antibiotika werden Zellklone isoliert, die die Plasmide mit dem Fusionsgen und dem Gen für die leichten Ketten enthalten (Transfektomas). Durch geeignete Nachweismethoden (BioDot; ELISA) werden solche Transfektomas identifiziert, die das Fusionsprotein der Formel A-Sp-E bestehend aus MAk-Fab-Teil, Linkerpolypeptid und Enzym sezernieren.

B) Eine Restriktionsschnittstelle A wird am 3'-Ende des Hinge Exons des Gens für die schweren Ketten des Immunglobulins eingeführt. Die Restriktionsschnittstelle A wird an der Stelle des Enzymgens eingeführt, an der die kodierende Nuldeotidsequenz für das reife Protein beginnt. Das Genfragment der schweren Ketten des Immunoglobulins mit den V_{H}-, C_{H}1- und Hinge-Exons wird über die Restriktionsschnittstelle A mit dem Enzymgen verknüpft.

Die Restriktionsstellen A werden so gesetzt, daß bei der Verknüpfung das Leseraster nicht gestört wird. Der Hinge-Teil des Antikörpers hat in dieser Konstruktion die Funktion des Polypeptidspacers.

Das Fusionsgen aus V_{H}-, CH 1-Hinge und Enzymgen wird in ein Expressionsplasmid kloniert, das für die Expression in eukaryontischen Zellen geeignet ist und einen Selektionsmarker trägt. Das Expressionsplasmid mit dem Fusionsgen wird zusammen mit einem Expressionsplasmid, das das zum Antikörper gehördende Leichte-Ketten-Gen enthält, in eukaryontische Expressionszellen transfiziert. Nach Selektion mit einem geeigneten Antibiotikum wurden Transfektomaklone identifiziert, die die Expressionsplasmide enthalten. Durch geeignete Nachweismethoden (BioDot, ELISA) werden solche Transfektomaklone identifiziert, die das Fusionsprotein der Formel II bestehend aus Antikörper und Enzym sezernieren.

Die Kopplung zwischen Enzym und Antikörper, dessen Fragment oder einem Biomolekül wird nach in der Literatur beschriebenen verfahren durchgeführt (A. H. Blair und T I. Ghose, I. Immunolog. Methods 59 (1983) 129-143; T I. Ghose et al. Methods in enzymology Vol. 93 (1983) 280-333).

Hierbei wird zunächst das Enzym über dessen Aminogruppe mittels succinimidyl-N-maleimido-alkylteiden-, cycloalkytiden- oder arylen-1-carboxylat funktionalisiert, wobei die Doppelbindung der Maleimidofunktion in eine Reaktion mit der HS-Gruppe des funktionalisierten Antikörpers, dessen Fragmentes oder der Biomoleküle unter Herausbildung einer Thioetherfunktion eingeht.

Zur Herstellung der Antikörper-Enzym-Konjugate können monoklonale Antikörper, die in EP-A-O 141 079 beschrieben werden, verwendet werden. Die Spezifität der Antikörper gegenüber tumor-assoziierten Antigenen wurde bereits am Tier- und Menschen mittels Immunszintigraphie und Immunhistochemie bewiesen.

Zur Herstellung der tumorspezifischen Enzym-Konjugate können nachfolgend genannte Enzyme aus der bezeichneten Quelle gemäß der angegebenen Literaturvorschrift gereinigt werden:
- alpha-Galactosidase aus humaner Leber Dean, K.G. & Sweeley, C.C. (1979), J. Biol. Chem. 254, 994-10000
- beta-Glucuronidase aus humaner Leber Ho, K.J. (1985) Biochim. Biophys. Acta 827, 197-206
- alpha-L-Fucosidase aus humaner Leber Dawson, G., Tsay, G. (1977) Arch. Biochem. Biophys. 184, 12-23
- alpha-Mannosidase aus humaner Leber Grabowski, G.A., Ikonne, J.U., Desnick, R.J. (1980) Enzyme 25, 13-25
- beta-Mannosidase aus humaner Plazenta Noeske, C., Mersmann, G. (1983) Hoppe Seylers Z Physiol. Chem. 364, 1645-1651
- alpha-Glucosidase aus humaner Magen-, Darmschleimhaut Asp, N.-G., Gudmand-Hoeyer, E., Christiansen, P.M., Dahlquist, A. (1974) Scand. J. Clin. Lab Invest. 33, 239-245
- beta-Glucosidase aus humaner Leber Daniels, L.B., Coyle, P.J., Chiao, Y-B., Glew, R.H. (1981) J. Biol. Chem. 256, 13004-13013
- beta-Glucocerebrosidase aus humaner Plazenta Furbish, F.S., Blair, H.E., Shiloach, J., Pentcheu, P.G., Brady, R.O. (1977) Proc. Natl. Acad. Sci, USA 74, 3560-3563
- alpha-N-Acetylglucosaminidase aus humaner Plazenta Roehrborn, W., von Figura, K. (1978) Hoppe Seylers Z physiol. Chem. 359,1353-1362
- beta-N-Acetylglucosaminidase aus humaner Amnionmembran Orlacchio, A., Emiliani, C., Di Renzo, G.C., Cosmi, E.V. (1986) Clin. Chim. Acta 159, 279-289
- alpha-N-Acetylgalactosaminidase gemäß Salvayre, R., Negre, A., Maret, A., Douste-Blazy, L. (1984) Pathol. Biol. (Paris) 32, 269-284.

Die glycolytische Aktivität der funktionalisierten tumorspezifischen Enzyme wurde in vergleichenden Untersuchungen mit p-Nitro-phenylglycosiden beim jeweiligen pH-Optimum ermittelt.

Gegenstand der Erfindung ist weiterhin eine Verpackung enthaltend ein erfindungsgemäßes Glycosyletoposid und ein funktionalisiertes tumorspezifisches Enzym-Konjugat in Kombination mit funktionalisierten tumorspezifischen Enzym-Konjugaten.

Zur Prüfung der Wirksamkeit einer kombinierten zeitlich versetzten Anwendung wurde Transplantat-Mäusen das funktionalisierte Enzym gegeben, gewartet bis zu dem Tag, an welchem der Plasmaspiegel des Enzym praktisch auf Null gesunken war, das Glycosyletoposid gegeben und beobachtet, ob ein Wachstumsstop und eine Regression eintraten.

### Beispiel 1

### Herstellung der Glycosylierungskomponente

### D-Glucuronsäurebenzylester (Verbinduna 1)

Zu einer Lösung von Natrium D-glucuronat (5 g, 23.13 mmol) in DMF (300 ml) wurde Benzylbromid (4.89 g, 28.59 mmol) zugegeben. Die Reaktionsmischung wurde 2 h bei 40° C, dann 16 h bei 80° C gerührt und in vacuo eingedampft. Der Rückstand wurde säulenchromatographisch über Kieselgel (130 g) mit 80:20:1 Chloroform-Methanol-Wasser gereinigt. Ausbeute: 4.87 g (74 %). Die Titelverbindung wurde mittels ¹³C-NMR charakterisiert.

### 1.2.3.4-Tetra-O-chloroacetyl-alpha und beta-D-glucuronsäure benzylester (Verbindung 2a und 2b)

D-Glucuronsäurebenzylester (3.80 g, 13.36 mmol) wurde in Dichlormethan (200 ml) suspendiert. Nach der Zugabe von Chloroacetylchlorid (7.90 g, 69.94 mmol) wurde die Mischung auf -30° C abgekuhlt und mit Pyridin (4.33 g, 54.74 mmol) gelöst in Dichlormethan (50 ml) versetzt. Die Reaktionsmischung wurde 16 h bei -30° C gerührt und anschließend mit Chloroacetylchlorid (7.90 g) und Pyridin (4.33 g) versetzt. Nach 16 h Rühren wurde die Mischung mit kaltem Dichlormethan (150 ml) versetzt und mit 5%igem Natriumcitrat-Puffer (pH 5, 60 ml x 2)) und Eiswasser (50 ml x 2) ausgewaschen. Das resultierende Produkt (7.92 g), das neben der Titelverbindung ca. 30 % 2,3,4- Tri-O-chloroacetyl-alpha,beta-D-glucuronsäure benzylester enthielt, wurde ohne weitere Reinigungsschritte in der nächsten Stufe eingesetzt.

### 2.3.4-Tri-O-chloroacetyl-alpha.beta-D-glucuronsäure benzylester (Verbindunc 3a, 3b)

Das Rohprodukt (7.92 g) der Verbindungen 2a/2b wurde in 3:1 Methanol-Chloroform (320 ml) gelöst und aminiertem Kieselgel (11.09 g) versetzt. Der Reaktionsansatz wurde 6 h bei Raumtemperatur gerührt und abfiltriert. Nach Abdampfen des Filtrates wurde der Rückstand chromatographisch über Kieselgel (220 g) mit 2:1 Petrolether-Ethylacetat gereinigt. Ausbeute: 4.94 g (72 % bezogen auf Verbindungen 2a/2b)

### 1-Desoxy-1-fluoro-alpha-D-glucuronsäure benzylester (Verbindung 4)

1-Desoxy-1-fluoro-alpha-D-glucuronsaure Natriumsalz (6.22 g, 28.51 mmol) wurde in DMF (350 ml) suspendiert und mit Benzylbromid (4.89 g, 28.59 mmol) versetzt. Die Reaktionsmischung wurde 24 h bei 60° C gerührt und anschließend eingedampft. Der Ruckstand wurde in 3:1 Chloroform-Methanol gelöst und mit Magnesiumsulfat (12 g) versetzt. Nach 2 h Rühren wurde die Suspension abfiltriert und das Filtrat wurde eingedampft. Der Rückstand wurde säulenchromatographisch über Kieselgel (160 g) mit 4:1 Dichlormethan-Aceton gereinigt.

Ausbeute: 5.95 g (73 %)

### 1-Desoxy-1-fluoro-2.3.4-tri-O-benzyl-alpha-D-glucuronsäure benzylester (Verbindung 5)

Verbindung 4 (5.0 g, 17.46 mmol) wurde in Dichlormethan (120 ml) suspendiert und bei 0° C Benzylbromid (9.85 g, 57.61 mmol) und Silberoxid (11.2 g) versetzt. Die Reaktionsmischung wurde 5 h bei 0° C, dann 28 h bei Raumtemperatur gerührt. Der Reaktionsansatz wurde abfiltriert, und das Filtrat wurde in vacuo eingedampft. Der Rückstand wurde chromatographisch über Kieselgel (240 g) mit 3:1 Petrolether-Ethylacetat gereinigt.

Ausbeute: 6.60 g (68 %)

### 1-Desoxy-1-fluoro-2,3,4-tri-O-chloroacetyl-alpha-D-glucuronsäure benzylester (Verbindung 6)

Verbindung 4 (5.0 g, 17.46 mmol) wurde in Dichlormethan (260 ml) supsendiert und bei -30° C mit Chloroacetylchlorid (9.86 g, 87.30 mmol) versetzt. Nach der Zugabe von 10:1 Dichlormethan-Pyridin (100 ml) wurde die Reaktionsmischung 18 h bei -30° C gerührt. Der Ansatz wurde mit kaltem Dichlormethan (80 ml) versetzt und mit Natriumcitrat-Puffer (pH 5.0, 80 ml x 3) und dann mit Wasser ausgewaschen. Die organische Phase wurde über Natriumsulfate getrocknet und eingedampft. Der Rückstand wurde säulenchromatographisch über Kieselgel (260 g) mit 5:1 Petrolether-Ethylacetat gereinigt.

Ausbeute: 7.58 g (92 %)

### 2,3.4.6-Tetra-O-chloroacetyl-alpha-D-galactopyranosyl fluorid (Verbindung

(182.15) alpha-D-Galactopyranosylfluorid (2.30 g, 12.62 mmol) wurde in trockenem Dichlormethan (100 ml) gelöst und bei -25° C mit Chloroacetylchlorid (9.0 g, 79.68 mmol) und 1:1 Dichlormethan-Triethylamin (55 ml) versetzt. Die Reaktionsmischung wurde 16 h gerührt und anschließend wurden Chloroacetylchlorid (9.0 g) und 1:1 Dichlormethan-Triethylamin (55 ml) zugegeben. Nach weiteren 24 h Rühren wurde der Reaktionsansatz mit Natriumcitrat-Puffer (pH 5.0, 50 ml x 3), dann mit Wasser ausgewaschen. Die organische Phase wurde getrocknet (Natriumsulfat) und in vacuo eingedampft. Der Rückstand wurde säulenchromatographisch über Kieselgel (300 g) mit 40:8:1 Dichlormethan-Petrolether-Ethylacetat gereinigt.
Ausbeute: 5.19 g (82 %)
alpha _{D} + 64.2° (c=1, Dichlormetham)

### 2.3.4.6-Tetra-O-benzyl-alpha-D-galactopyranosyl fluorid (Verbindung 8)

alpha-D-Galactopyranosyl fluorid (2.30 g, 12.62 mmol) wurde in trockenem DMF (40 ml) gelöst und bei -20⁰ C mit 171.04 Benzylbromid (12.95 g, 75.72 mmol) und Silberoxid (10 g) versetzt. Die Reaktionsmischung wurde 5 h bei -20° C, dann 24 h bei Raumtemperatur gerührt. Anschließend wurden die Salze abfiltriert und das Filtrat in vacuo eingedampft. Der Ruckstand wurde säulenchromatographisch über Kieselgel (350 g) mit 15:1 Petrolether-Ethylacetat gereinigt.

Ausbeute: 5.20 g (76 %)

### Beispiel 2

### Glycosidsynthese

### 4'-O-Demethyl-4-O-(2.3-di-O-chloroacetyl-4.6-O-ethylide-n-beta-D-glucpyvranosyl-4-epi-podophyllotoxin(Verbindung 9)

4'-O-Benzyloxycarbonyl-4-O-demethyl-4-O-(2,3-di-O-chloroacetyl-4,6- O-ethyliden-beta-D-glucopyranosyl)-4-epi- podophyllotoxin (10 g, 11.42 mmol) wurde in 2:1 Ethylacetat-Methanol (200 ml) in der Gegenwart von 10 % Pd/C (5.0 g) 2 h hydriert. Die Reaktionsmischung wurde abfiltriert, und das Filtrat wurde in vacuo eingedampft. Der Rückstand wurde über eine Kieselgelschicht (50 g) filtriert. Das resultierende Produkt wurde mit Methanol-Ethylacetat kristallisiert.

### Ausbeute: 7.50 g (88.6 %); Schmp. 201-203° C; alpha _{D} -73.4° (c=1, Chloroform)

### 4'-O-Demethyl-4-O-(di-O-chloroacetyl-4.6-O-ethyliden-beta-D-glucopyranosy)-4-epi-4'-O- (2.3.4-tri-O-benzyl-beta-D-glucopyranosyl)- uronsäurebenzylester -podophyllotoxin (Verbindung 9)

1-Fluoro-glucuronsäure benzylester (Verbindung 5, 4.23 g, 7.60 mmol) und 2",3"-Di-O-chloroacetyl-etoposid (Verbindung 9, 5.63 g, 7.60 mmol) wurden in Dichlormethan (220 ml) gelöst und mit Molekularsieb 4 A (10 g) versetzt. Die Reaktionsmischung wurde bei -40° C mit BF₃- Ether (2.5 ml) versetzt und anschließend 20 h bei -30° C gerührt. Nach der Zugabe von Triethylamin (7.0 ml) wurde die Mischung abfiltriert. Das Filtrat wurde mit Citrat-Puffer (pH 5, 80 ml x 3) und Wasser (120 ml x 3) gewaschen, getrocknet (Natriumsulfat) und in vacuo eingedampft. Der Rückstand wurde säulenchromatographisch über Kieselgel (360 g) mit 5:5:1 Dichlormethan-Petrolether-Aceton gereinigt.

Ausbeute: 6.49 g (67 %)

### 4'-O-Demethyl-4-O-(di-O-chloroacetyl-4,6-0-ethyliden-be ta-D-glucopyranosyl)-4-epi-4'-O-(2.3.4.6-tetra-O-benzyl alpha-und beta-D-aalactopyranosyl)-podophyllotoxin (Verbinduna 10a und 10b)

Tetra-O-benzyl-galactopyranosyl fluorid (Verbindung 8, 3.60 g, 6.65 mmol) und Etoposid-Derivat (Verbindung 9, 4.93 g, 6.65 mmol) wurden in Dichlormethan (250 ml) gelöst. Nach der Zugabe von Molekularsieb 4 A (7.2 g) wurde die Reaktionsmischung auf -40° C abgekühlt und 30%iger BF₃-Ether (2.2 ml) wurde zugetropft. Die Mischung wurde 20 h bei -30° C gerührt, anschließend mit Triethylamin (5.5 ml) versetzt und abfiltriert. Das Filtrat wurde mit Citrat-Puffer (75 ml x 3, pH 5) und Wasser (70 ml x 2) gewaschen, über Natriumsulfat getrocknet und eingedampft. Der Rückstand wurde säulenchromatographisch über Kieselgel (370 g) mit 5:5:1 Petrolether-Dichlormethan-Aceton vorgereinigt. Die weitere säulenchromatographische Trennung lieferten die Titelverbindungen 10a (alpha-Glycosid: 4.36 g (52 %)) und 10b (beta- Glycosid: 1.42 g (17 %)).

### Beispiel 3

### Entblockierungsreaktion bei Glycosyletoposiden

### 4'-O-Demethyl-4-epi-4-O-(4.6-O-ethyliden-beta-D-glucopyranosyl-)-4'-O- (2.3.4-tri-O-benzyl-beta-D-glucopyranosyl)-uronsäure benzylester podophyllotoxin (Verbindung 11)

Glucuronid-Derivat (Verbindung 9, 4.56 g, 3.57 mmol) wurde in 4:1 Methanol-Chloroform (120 ml) gelöst und mit DOWEX 1 x8 (6.2 g) versetzt. Der Reaktionsansatz wurde 3 h bei Raumtemperatur gerührt und anschließend abfiltriert. Das Filtrat wurde mit Chloroform (150 ml) versetzt und mit Magnesiumsulfat (10 g) verrührt. Nach Abfiltrieren der Salze wurde das Filtrat in vacuo eingedampft. Der Rückstand wurde säulenchromatographisch über 200 g Kieselgel mit 5:2:1 Dichlormethan-Petrolether-Aceton gereinigt.

### Ausbeute: 3.29 g (82 %)

### 4'-O-Demethyl-4-epi-4-O-(4.6-O-ethyliden-beta-D-glucopyranosyl)-4'-O- (beta-D-glucopyranosyl)-uronsäure podophyllotoxin (Verbindung 12)

Deacyliertes Glucuronid-Derivat (Verbindung 11, 3.62 g, 3.22 mmol) wurde in 4:1 Methanol-Ethylacetat (180 ml) gelöst und in der Gegenwart von 10 % Pd/C (2.76 g) 20 h hydriert. Nach Abfiltrieren des Katalysators wurde das Filtrat in vacuo eingedampft. Der Rückstand wurde säulenchromatographisch über RP-18 Kieselgel mittels Methanol-Hexan (Gradient) gereinigt.

### Ausbeute: 1.82 g (74 %)

### 4'-O-Demethyl-4-epi-4-O-(4.6-0-ethyliden-beta-D-glucopyranosyl)-4'-0-(2.3.4.6-tetra-0-benzyl-alpha-D-galactopyranosyl)-podophyllotoxin (Verbinduna 13)

Podophyllotoxin-galactopyranosid (Verbindung 10a, 3.0 g, 2.37 mmol) wurde in 3:1 Methanol-Chloroform gelöst und mit Dowex 1 x8 versetzt. Die Reaktionsmischung wurde 3 h bei Raumtemperatur gerührt und abfiltriert. Das Filtrat wurde in vacuo eingedampft. Der in Chloroform gelöste Rückstand wurde mit Phosphat-Puffer (pH 7, 30 ml x 3), dann mit Wasser gewaschen. Die organische Phase wurde über Natriumsulfat getrocknet und eingedampft. Der Rückstand wurde säulenchromatographisch über Kieselgel (85 g) mit 5:3:1 Dichlormethan-Petrolether-Aceton gereinigt.

### Ausbeute: 2.27 g (86 %)

### 4'-O-Demethyl-4-eni-4'-O-(alpha-D-galactopyranosyl)-4-O-(4.6-O-ethyliden-beta-D-glucopyranosyl)-podophyllotoxin (Verbindung 14)

Dacyliertes Podophyllotoxin-galactopyranosid (Verbindung 13, 2.0 g, 1.80 mmol) wurde in 3:1 Methanol-Ethylacetat gelöst und in der Gegenwart von 10%ige Pd/C (2.5 g) 24 h hydriert. Der Ansatz wurde mit Magnesiumsulfat verrührt und abfiltriert. Das Filtrat wurde in vacuo eingedampft. Der Rückstand wurde säulenchromatographisch über RP-18 Kieselgel mittels Methanol-Ethylacetat (Gradient) gereinigt.

Ausbeute: 1.37 g ( 72 %)

### Beispiel 4

### a) Spaltung von 4'-O-alpha-D-Galactopyranosyl-etoposid mit alpha-Galactosidase (aus grünen Kaffeebohnen)

4'-O-alpha-D-Galactopyranosyl-etoposid wurde in einer Konzentration von 1 mg/ml in 20 mM Natriumphosphatpuffer, pH 5, gelöst, mit 0.3U/ml alpha-Galactosidase (green coffee beans; sigma Co.; 1 U = Spaltung von 1 µmol p-Nitrophenyl-alpha-D-galactosid per Minute bei pH 6.5 und 25 Grad) versetzt und bei 37 Grad inkubiert. Der Abbau von 4'-O-alpha-D-Galactopyranosyl-etoposid und das Auftauchen von freiem Etoposid wurden mittels HPLC untersucht. Die Halbwertszeit betrug 15 Minuten.

### b) S^{p}altuna von 4'-O-alpha-D-Galactopyanosyl-etoposid mit alpha-Galactosidase A (aus humaner Plazenta)

4'-O-alpha-D-Galactopyranosyl-etoposid wurde in einer Konzentration von 107 bzw. 10.7 µg/ml in 20 mM Natriumphosphatpuffer, ph %, gelöst, mit 0.36 U/ml alpha-Galactosidase A (isoliert aus humaner Plazenta; 1 U = Spaltung von 1 µmol 4-Methumbelliferyl-alpha-D-galactosid per Minute bei pH 5 und 37 Grad) versetzt und bei 37 Grad inkubiert. Der Abbau von 4'-O-alpha-D-Galacto-pyranosyl-etoposid und das Auftauchen von freiem Etoposid wurden mittels HPLC untersucht. Die Halbwertszeit betrug 4 bzw. 7 Stunden.

### Beispiel 11

### A. Glycosylierung von Etoposiden

### Allgemeine Vorschrift:

Zu einer Lösung von 2",3"-Di-O-chloroacetyl-etoposid (0.67 mmol) und Glycosylhalogenid (Bromid oder Chlorid, 1.2 mmol) in Dichlormethan (50 mL) wurden Molekularsieb (1.4 g), Silber carbonat (0.857 g) und Silberperchlorat z bei -20°C zugegeben und die Reaktionsmischung wurde 60 h unter Lichtausschluß gerührt. Die Mischung wurde mit Dichlormethan (50 mL) versetzt und abfiltriert. Das Filtrat wurde mit Wasser ausgewaschen und über Magnesium sulfat getrocknet. Der Rückstand wurde chromatgraphiert, wobei die alpha- bzw. β-O-glycosidisch verknüpfte Verbindungen erhalten wurden.

### 2",3"-Di-O-chloroacetyl-4'-O-(2,3,4,6-tetra-O-benzyl-alpha-D-galactopyranosyl)-etoposid

Die Titelverbindungen wurden ausgehend von 2,3,4,6-tetra-O-benzyl-alpha-D-galactopyranosyl-chlorid (bzw.-bromid) und 2",3"-Di-O-chloroacetyl-etoposid nach der oben genannten Vorschrift hergestellt.

### alpha-Glycosid [alpha]_{D} +11.8° (c=1, Chloroform)

### 2",3"-Di-O-chloroacetyl-4'-O-(2,3,4,6-tetra-O-benzyl-ß-D-galactopyranosyl)-etoposid

Die Titelverbindungen wurden ausgehend von 2,3,4,6-tetra-O-benzyl-alpha-D-galactopyranosyl-bromid und 2",3"-Di-O-chloroacetyl-etoposid nach der oben genannten Vorschrift hergestellt.

### β-Glycosid [alpha]_{D} -39.8° (c=1, Chloroform)

### 2",3"-Di-O-chloroacetyl-4'-0-(2,3,4,6-tetra-O-benzyl-alpha-D-glucopyranosyl)-etoposid

Die Titelverbindungen wurden ausgehend von 2,3,4,6-tetra-O-benzyl-alpha-D-glucopyranosyl-chlorid (bzw.-bromid) und 2",3"-Di-O-chloroacetyl-etoposid nach der oben genannten Vorschrift hergestellt.

### alpha-Glycosid [alpha]_{D} +16.2° (c=1, Chloroform)

### 2",3"-Di-O-chloroacetyl-4'-O-(2,3,4,6-tetra-O-benzyl-β-D-glucopyranosyl)-etoposid

Die Titelverbindungen wurden ausgehend von 2,3,4,6-tetra-O-benzyl-alpha-D-glucopyranosyl-bromid und 2",3"-Di-O-chloroacetyl-etoposid nach der oben genannten Vorschrift hergestellt.

### ß-Glycosid [alpha]_{D} -44.5° (c=1, Chloroform)

### 2",3"-Di-O-chloroacetyl-4'-O-(2,3,4,6-tetra-O-benzyl-β-D-glucuronyl)-etoposid

Die Titelverbindungen wurden ausgehend von Benzyl 2,3,4-tri-O-benzyl-1-chloro (bzw. -bromo)-1-deoxy-alpha-D-glucupyranuronat und 2",3"-Di-O-chloro-acetyl-etoposid nach der oben genannten Vorschrift hergestellt.

### β-Glycosid [alpha]_{D} -52.4° (c=1, Chloroform)

### B. Entblockierung von Cloroacetyl-Schutzgruppe in Glycosyl-etoposiden

### Allgemeine Vorschrift:

Eine Mischung aus einem 2",3"-di-O-chloroacetylierten Glycosyl-etoposid (0.75 mmol) und lonenaustauscher Dowex 1X8 (3.0 g) in 3:2 Methanol-Dichloromethan (200 mL) wurde 1 h bei Rautemteratur gerührt. Nach Abfiltrieren des Harzes wurde das Filtrat mit Phosphat-Puffer (pH 6) gewaschen, getrocknet (Natrium sulfat) und in vacuo eingedampft.

Der Rückstand wurde säulenchromatographisch gereinigt. Folgenden Verbindungen wurden hergestellt:
4'-O-(2,3,4,6-Tetra-O-benzyl-alpha-D-galactopyranosyl)-etoposid alpha-Glycosid [alpha]_{D} +6.0° (c=1, Chloroform)
4'-O-(2,3,4,6-Tetra-O-benzyl-b-D-galactopyranosyl)-etoposid
4'-O-(2,3,4,6-Tetra-O-benzyl-alpha-D-glucopyranosyl)-etoposid alpha-Glycosid [alpha]_{D} +14.9° (c=1, Chloroform)
14'-0-(2,3,4,6-Tetra-O-benzyl-ß-D-glucopyranosyl)-etoposid β-Glycosid [alpha]_{D} -53° (c=1.04, Chloroform)
4'-O-(2,3,4,6-Tetra-O-benzyl-[3-D-glucuronyl)-etoposid

### Hydrogenolytische Abspaltung von Benzyl-Schutzgruppen in Glycosyl-Etoposiden

### Allgemeine Vorschrift:

Eine Mischung eines benzylierten Glycosyl-etoposides (0.64 mmol) in Eisessig (10 mL) wurde in der Gegenwart von 10%-Pd/C (1.0 g) 24 h hydriert. Nach Abfiltrieren des Katalysators wurde das Filtrat in vacuo bei ca. 0°C eingedampft. Der Rückstand wurde säulenchromatographisch am Kieselgel gereinigt, wobei folgenden Glycosyl-etoposide gewonnen wurden:
4'-O-alpha-D-Galactopyranosyl)-etoposid alpha-Glycosid [alpha]_{D} +6.2° (c=1, Chloroform)
4'-0- β-D-Galactopyranosyl)-etoposid β-Glycosid [alpha]_{D} -73.1° (c=1, Chloroform)
4'-O-alpha-D-Glucopyranosyl)-etoposid alpha-Glycosid [alpha]_{D} +19.2° (c=₁, Chloroform)
4'-O-β-D-Glucopyranosyl)-etoposid β-Glycosid [alpha]HDY -76.6° (c=0.9, Chloroform)
4'-O-ß-D-glucuronyl)-etoposid

## Patentansprüche (Patentansprüche für folgende(n) Vertragsstaat(en) : AT, BE, CH, DE, DK,FR, GB, IT, LI, LU, NL, SE)

1. Verfahren zur Herstellung einer Verbindung der Formel I, 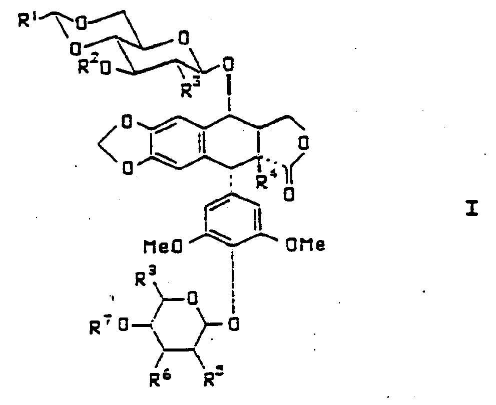 worin
R¹ eine Methyl-, Benzyl- oder 2-Thienylgruppe,
R² ein Wasserstoffatom,
R³ eine Hydroxy-, Amino- oder Dimethylaminogruppe,
R⁴ ein Wasserstoffatom oder eine Methylgruppe,
R⁵ ein Wasserstoffatom, eine Hydroxygruppe, eine Amino- oder Acetylaminogruppe,
R⁶ eine Hydroxygruppe oder eine Aminogruppe,
_{R}⁷ ein Wasserstoffatom,
R⁸ eine Methyl- oder Hydroxymethylgruppe oder eine Carboxygruppe oder eine über Methylenoxy-Gruppe gebundene Acyl-Schutzgruppe oder eine Benzyloxycarbonylgruppe,
wobei die Acylschutzgruppe eine Acetyl-, Mono-, Di- oder Trihalogenacetylgruppe mit Halogen, Fluor oder Chlor ist, sind,
dadurch gekennzeichnet, daß man eine Etoposid-Verbindung der Formel V, 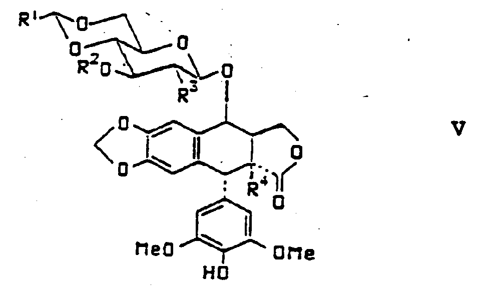 worin
R¹ eine Methyl-, Benzyl- oder 2-Thienylgruppe,
R² ein Wasserstoffatom, eine Acyl-, oder eine Tri-C₁-C₄-alkylsilyl-Schutzgruppe,
R³ eine Hydroxygruppe, eine über Sauerstoff gebundene Acyl- oder Tri-C1-C4-alkrylsilyl-Schutzgruppe, Acetylamino-, Benzyloxycarbonylamino- oder Dimethylaminogruppe und
R⁴ ein Wasserstoffatom oder eine Methylgruppe darstellen,
mit einer Kohlenhydrat-Komponente der Formel VI, 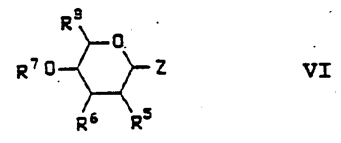 worin
R⁵ ein Wasserstoffatom, eine Hydroxygruppe, eine über ein Sauerstoffatom gebundene Acyl-Schutzgruppe, oder Benzyloxycarbonylamino-, Azido- oder Acetylaminogruppe,
R⁶ eine über ein Sauerstoffatom gebundene Acyl-Schutzgruppe, eine Benzyloxycarbonylamino- oder Azidogruppe,
R⁷ eine Acyl-Schutzgruppe,
R⁸ eine Methylgruppe, Methylenoxy-Acyl-Schutzgruppe oder eine Benzyloxycarbonylgruppe und
Z ein Halogenatom, bevorzugt Fluor, Chlor oder Brom, eine Hydroxygruppe, eine Tri-C₁-C₄-alkylsilyloxy-Gruppe oder eine über Sauerstoffatomgebundene Acyl-Schutzgruppe,
wobei die Acyl-Schutzgruppe eine Acetyl-, Mono-, Di- oder Trihalogenacetylgruppe, bevorzugt mit Halogenatom Fluor oder Chlor ist, darstellen, in der Gegenwart eines Promotors und gegebenenfalls eines Säurefängers oder Trockenmittels in einem Lösungsmittel bei -50° C bis 60° C zu einem 4'-O-Glycosyl-etoposid-Derivat der Formel I, worin alle Reste R¹ bis R⁸ ihre Bedeutung, wie oben definiert, beibehalten, umsetzt, und in diesen Verbindungen die vorhandenen Schutzgruppen hydrogenolytisch oder hydrolytisch abspaltet und gegebenenfalls eine von den entstandenen Amino-gruppen-enthaltenden Verbindungen mittels reduktiver Alkylierung in eine weitere Dimethylaminogruppe-enthaltende Verbindung der Formel I umsetzt.

2. Verfahren nach Anspruch 1 , dadurch gekennzeichnet, daß die Acyl-Schutzgruppen eine Acetyl-, Chloroacetyl-oder Trifluoracetylgruppe ist.

3. 4'-O-Glycosyl-Etoposid der allgemeinen Formel I, worin
R¹ eine Methyl-, Benzyl- oder 2-Thienylgruppe,
R² ein Wasserstoffatom, eine Acyl- oder Tri-C1-C4-alkylsilyl-Schutzgruppe,
R³ eine Hydroxygruppe, eine über ein Sauerstoffatom gebundene Acyl-oder Tri-C1-C4-alkylsilyl-Schutzgruppe, eine Amino-, Acetylamino-, Benzyloxycarbonylamino- oder Dimethylaminogruppe,
R⁴ ein Wasserstoffatom oder eine Methylgruppe,
R⁵ ein Wasserstoffatom, eine Hydroxygruppe, eine über ein Sauerstoffatom gebundene Acyl- oder Tri-C₁-C₄- alkylsilyl-Schutzgruppe, eine Amino-, Benzyloxycarbonylamino-, Azido- oder Acetylaminogruppe,
R⁶ eine Hydroxygruppe, eine über ein Sauerstoffatom gebundene Acyl-, oder Tri-C₁-C₄-alkylsilyl-Schutzgruppe, eine Amino-, Benzyloxycarbonylamino- oder Azidogruppe,
R⁷ ein Wasserstoffatom, eine Acyl- oder Tri-C1-C4-alkylsilyl-Schutzgruppe und
R⁸ eine Methyl-, Hydroxymethyl- oder eine über Methylenoxy-Gruppe gebundene Acyl-Schutzgruppe oder eine Benzyloxycarbonylgruppe, wobei die Acylschutzgruppe eine Acetyl-, Mono-, Di- oder Trihalogenacetylgruppe mit Halogen, Fluor oder Chlor ist,
darstellen.

4. Verbindung nach Anspruch 3, worin
R¹ eine Methyl, Benzyl oder 2-Thienylgruppe,
R² ein Wasserstoffatom, eine Acetyl-, Chloroacetyl- oder eine Tri-C₁-C₄-alkylsilyl-Schutzgruppe,
R³ eine Hydroxygruppe, eine über ein Sauerstoffatom gebundene Acetyl-, Chloroacetyl- oder Tri-C₁-C₄- alkylsilyl-schutzgruppe, eine Amino-, Acetylamino-, Benzyloxycarbonylamino- oder Dimethylaminogruppe,
R⁴ ein Wasserstoffatom oder eine Methylgruppe,
R⁵ ein Wasserstoffatom, eine Hydroxygruppe oder eine über ein sauerstoffatom gebundene Acetyl-, Chloroactyl- oder Tri-C₁-C₄-alkylsilyl-Schutz gruppe, eine Amino-, Benzyloxycarbonylamino-, Azido- oder Acetylaminogruppe,
R⁶ eine Hydroxygruppe, eine über ein Sauerstoffatom gebundene Acetyl-, Chloroacetyl- oder Tri-C₁-C₄- alkylsilyl-Schutzgruppe, eine Amino-, Benzyloxycarbonylamino- oder Azidogruppe,
R⁷ ein Wasserstoffatom, eine Acetyl-, Chloroacetyl- oder Tri-C₁-C₄-alkylsilyl-Schutzgruppe und
R⁸ eine Methyl-, Hydroxymethyl-, Acetyloxy- oder Chloroacetyloxymethylgruppe oder eine Benzyloxycarbonylgruppe
sind.

5. Arzneimittel enthaltend eine Verbindung nach Anspruch 3 und ein funktionalisiertes tumor-spezifisches Enzym der Formel II 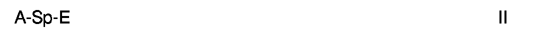 worin A einen tumorspezifischen Antikörper oder ein Fragment davon oder ein in einem Tumor sich anreicherndes Biomolekül darstellt, E eine nicht oder wenig immunogene Glykosidase bedeutet und Sp (Spacer) eine bifunktionelle Sulfid- oder Disulfid enthaltende Gruppe der Formel III oder IV 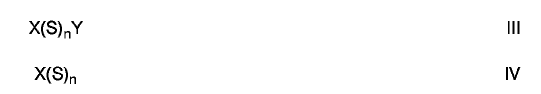 oder einen Polypeptid-Spacer darstellt, worin
X oder Y -CO-R⁹⁻(N-succinimido) - oder -C (=R¹⁰)-CH₂-CH₂- mit R⁹ -CH₂-CH₂-, 1,4-Cyclohexyliden, 1,3 oder 1,4-Phenylen oder Methoxycarbonyl- oder Chloro-phen-1,4-ylen und R¹⁰ O oder NH, und weiterhin
Y -C(=R¹⁰)-CH₂-CH₂-, wobei R¹⁰ die angegebene Bedeutung hat, und
n 1 oder 2
bedeutet.

6. Mittel nach Anspruch 5, dadurch gekennzeichnet, daß A-Sp-E gentechnisch herstellbar ist; wobei A und E die Bedeutung wie in Anspruch 5 haben und Sp ein Oligo- oder Polypeptid darstellt.

7. Funktionalisiertes tumorspezifisches Enzym der Formel (II) wie in Anspruch 5 definiert enthaltend eine ß-Glucuronidase.

## Patentansprüche (Patentansprüche für folgende(n) Vertragsstaat(en) : ES, GR)

1. Verfahren zur Herstellung einer Verbindung der Formel I, 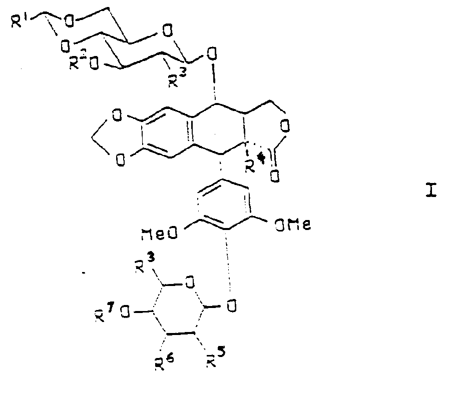 worin
R¹ eine Methyl-, Benzyl- oder 2-Thienylgruppe,
R² ein Wasserstoffatom,
R³ eine Hydroxy-, Amino- oder Dimethylaminogruppe,
R⁴ ein Wasserstoffatom oder eine Methylgruppe,
R⁵ ein Wasserstoffatom, eine Hydroxygruppe, eine Amino- oder Acetylaminogruppe,
R⁶ eine Hydroxygruppe oder eine Aminogruppe,
_{R}⁷ ein Wasserstoffatom,
R⁸ eine Methyl- oder Hydroxymethylgruppe oder eine Carboxygruppe oder eine über Methylenoxy-Gruppe gebundene Acyl-Schutzgruppe oder eine Benzyloxycarbonylgruppe,
wobei die Acylschutzgruppe eine Acetyl-, Mono-, Di- oder Trihalogenacetylgruppe mit Halogen, Fluor oder Chlor ist, sind,
dadurch gekennzeichnet, daß man eine Etoposid-Verbindung der Formel V, 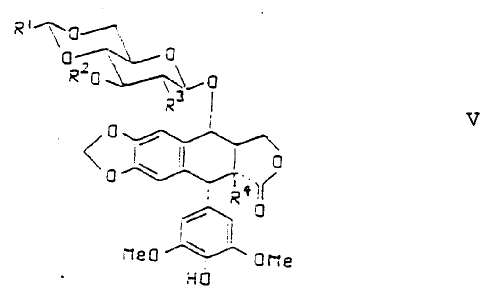 worin
R¹ eine Methyl-, Benzyl- oder 2-Thienylgruppe,
R² ein Wasserstoffatom, eine Acyl-, oder eine Tri-C₁-C₄-alkylsilyl-Schutzgruppe,
R³ eine Hydroxygruppe, eine über Sauerstoff gebundene Acyl- oder Tri-C₁-C₄-alkylsilyl-Schutzgruppe, Acetylamino-, Benzyloxycarbonylamino- oder Dimethylaminogruppe und
R⁴ ein Wasserstoffatom oder eine Methylgruppe darstellen,
mit einer Kohlenhydrat-Komponente der Formel VI, 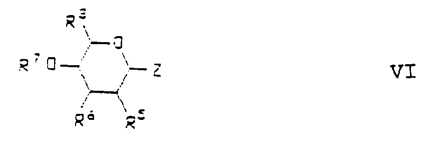 worin
R⁵ ein Wasserstoffatom, eine Hydroxygruppe, eine über ein Sauerstoffatom gebundene Acyl-Schutzgruppe, oder Benzyloxycarbonylamino-, Azido- oder Acetylaminogruppe,
R⁶ eine über ein Sauerstoffatom gebundene Acyl-Schutzgruppe, eine Benzyloxycarbonylamino- oder Azidogruppe,
_{R}⁷ eine Acyl-Schutzgruppe,
R⁸ eine Methylgruppe, Methylenoxy-Acyl-Schutzgruppe oder eine Benzyloxycarbonylgruppe und
Z ein Halogenatom, bevorzugt Fluor, Chlor oder Brom, eine Hydroxygruppe, eine Tri-C₁-C₄-alkylsilyloxy-Gruppe oder eine über Sauerstoffatomgebundene Acyl-Schutzgruppe,
wobei die Acyl-Schutzgruppe eine Acetyl-, Mono-, Di- oder Trihalogenacetylgruppe, bevorzugt mit Halogenatom Fluor oder Chlor ist, darstellen, in der Gegenwart eines Promotors und gegebenenfalls eines Säurefängers oder Trockenmittels in einem Lösungsmittel bei -50° C bis 60° C zu einem 4'-O-Glycosyl-etoposid-Derivat der Formel I, worin alle Reste R¹ bis R⁸ ihre Bedeutung, wie oben definiert, beibehalten, umsetzt, und in diesen Verbindungen die vorhandenen Schutzgruppen hydrogenolytisch oder hydrolytisch abspaltet und gegebenenfalls eine von den entstandenen Aminogruppen-enthaltenden Verbindungen mittels reduktiver Alkylierung in eine weitere Dimethylaminogruppe-enthaltende Verbindung der Formel I umsetzt.

2. Verfahren nach Anspruch 2, dadurch gekennzeichnet, daß die Acyl-Schutzgruppen eine Acetyl-, Chloroacetyl-oder Trifluoracetylgruppe ist.

3. Verfahren nach Anspruch 1, dadurch gekennzeichnet, daß
R¹ eine Methyl-, Benzyl- oder 2-Thienylgruppe,
R² ein Wasserstoffatom, eine Acyl- oder Tri-C₁-C₄-alkylsilyl-Schutzgruppe,
R³ eine Hydroxygruppe, eine Acyl- oder Tri-C₁-C₄-alkylsilyl-Schutzgruppe, eine Amino-, Acetylamino-, Benzyloxycarbonylamino- oder Dimethylaminogruppe,
R⁴ ein Wasserstoffatom oder eine Methylgruppe,
R⁵ ein Wasserstoffatom, eine Hydroxygruppe, eine über ein Sauerstoffatom gebundene Acyl- oder Tri-C₁-C₄- alkylsilyl-Schutzgruppe, eine Amino-, Benzyloxycarbonylamino-, Azido- oder Acetylaminogruppe,
R⁶ eine Hydroxygruppe, eine über ein Sauerstoffatom gebundene Acyl-, oder Tri-C₁-C₄-alkylsilyl-Schutzgruppe, eine Amino-, Benzyloxycarbonylamino- oder Azidogruppe,
R⁷ ein Wasserstoffatom, eine Acyl- oder Tri-C1-C4-alkylsilyl-Schutzgruppe und
R⁸ eine Methyl-, Hydroxymethyl- oder eine über Methylenoxy-Gruppe gebundene Acylschutzgruppe oder eine Benzyloxycarbonylgruppe, wobei die Acylschutzgruppe eine Acetyl-, Mono-, Di- oder Trihalogenacetylgruppe mit Halogen, Fluor oder Chlor ist.

4. Verfahren nach Anspruch 1, dadurch gekennzeichnet, daß
R¹ eine Methyl, Benzyl oder 2-Thienylgruppe,
R² ein Wasserstoffatom, eine Acetyl-, Chloroacetyl- oder eine Tri-C₁-C₄-alkylsilyl-Schutzgruppe,
R³ eine Hydroxygruppe, eine über ein Sauerstoffatom gebundene Acetyl-, Chloroacetyl- oder Tri-Cᵢ-C₄-alkylsi- lyl-Schutzgruppe, eine Amino-, Acetylamino-, Benzyloxycarbonylamino- oder Dimethylaminogruppe,
R⁴ ein Wasserstoffatom oder eine Methylgruppe,
R⁵ ein Wasserstoffatom, eine Hydroxygruppe oder eine über ein Sauerstoffatom gebundene Acetyl-, Chloroacetyl- oder Tri-C₁-C₄-alkylsilyl-Schutz gruppe, eine Amino-, Benzyloxycarbonylamino-, Azido- oder Acetylaminogruppe,
R⁶ eine Hydroxygruppe, eine über ein Sauerstoffatom gebundene Acetyl-, Chloroacetyl- oder Tri-C₁-C₄-alkylsi- lyl-Schutzgruppe, eine Amino-, Benzyloxycarbonylamino- oder Azidogruppe,
R⁷ ein Wasserstoffatom, eine Acetyl-, Chloroacetyl- oder Tri-C₁-C₄-alkylsilyl-Schutzgruppe und
R⁸ eine Methyl-, Hydroxymethyl-, Acetyloxy- oder Chloroacetyloxymethylgruppe oder eine Benzyloxycarbonylgruppe
sind.

5. Verfahren zur Herstellung eines Arzneimittels, dadurch gekennzeichnet, daß eine Verbindung nach Anspruch 3 und ein funktionalisiertes Enzym der Formel II 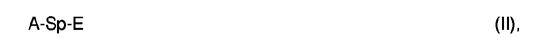 worin A einen tumorspezifischen Antikörper oder ein Fragment davon oder ein in einem Tumor sich anreicherndes Biomolekül darstellt, E eine nicht oder wenig immunogene Glykosidase bedeutet und Sp (Spacer) eine bifunktionelle Sulfid- oder Disulfid enthaltende Gruppe der Formel III oder IV 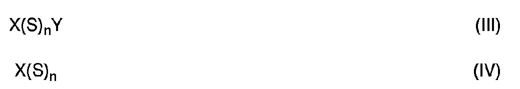 oder einen Polypeptid-Spacer darstellt, worin
X oder Y -CO-R⁹⁻(N-succinimido) - oder -C (=R¹⁰)-CH₂-CH₂- mit R⁹ -CH₂-CH₂-, 1,4-Cyclohexyliden, 1,3 oder 1,4-Phenylen oder Methoxycarbonyl- oder Chloro-phen-1,4-ylen und R¹⁰ O oder NH, und weiterhin
Y -C(=R¹⁰)-CH₂-CH₂-, wobei R¹⁰ die angegebene Bedeutung hat, und
n 1 oder 2 bedeutet
in einer geeigneten galenischen Form zusammengebracht werden.

6. Verfahren nach Anspruch 5, dadurch gekennzeichnet, daß A-Sp-E gentechnisch herstellbar ist, wobei A und E die Bedeutung wie in Anspruch 5 haben und Sp ein Oligo- oder Polypeptid darstellt.

7. Verfahren nach Anspruch 5, dadurch gekennzeichnet, daß das funktionalisierte tumorspezifische Enzym der Formel wie in Anspruch 5 definiert eine β-Glucuronidase enthält.

## Claims (Claims for the following Contracting State(s) : AT, BE, CH, DE, DK, FR, GB, IT, LI, LU, NL, SE)

1. A process for the preparation of a compound of the formula I 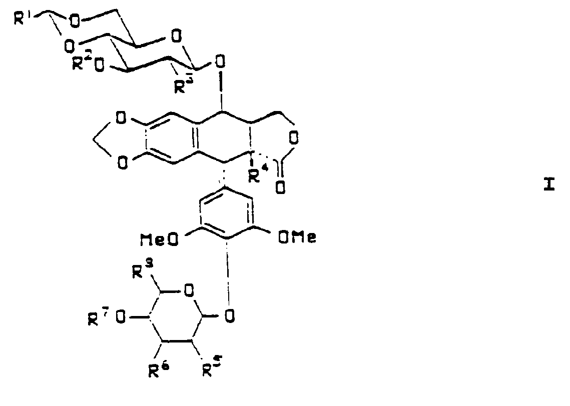 in which
R¹ is a methyl, benzyl or 2-thienyl group,
R² is a hydrogen atom,
R³ is a hydroxyl, amino or dimethylamino group,
R⁴ is a hydrogen atom or a methyl group,
R⁵ is a hydrogen atom, a hydroxyl group, an amino or acetylamino group,
R⁶ is a hydroxyl group or an amino group,
R⁷ is a hydrogen atom,
R⁸ is a methyl or hydroxymethyl group or a carboxyl group or an acyl protective group which is bonded via a methyleneoxy group, or a benzyloxycarbonyl group,
where the acyl protective group is an acetyl, mono-, di- or trihalogenoacetyl group with halogen being fluorine or chlorine,
which comprises reacting, in the presence of a promoter and, where appropriate, of an acid trap or drying agent in a solvent at -50°C to 60°C, an etoposide compound of the formula V 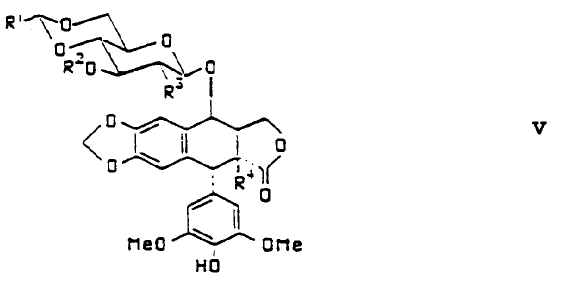 in which
R¹ is a methyl, benzyl or 2-thienyl group,
R² is a hydrogen atom, an acyl or a tri-C₁-C₄-alkylsilyl protective group,
R³ is a hydroxyl group, an acyl or tri-C₁-C₄-alkylsilyl protective group which is bonded via oxygen, or acetylamino, benzyloxycarbonylamino or dimethylamino group and
R⁴ is a hydrogen atom or a methyl group,
with a carbohydrate component of the formula VI 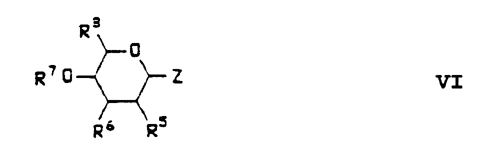 in which
R⁵ is a hydrogen atom, a hydroxyl group, an acyl protective group which is bonded via an oxygen atom, or benzyloxycarbonylamino, azido or acetylamino group,
R⁶ is an acyl protective group which is bonded via an oxygen atom, or a benzyloxycarbonylamino or azido group,
R⁷ is an acyl protective group,
R⁸ is a methyl group, methyleneoxy-acyl protective group or a benzyloxycarbonyl group and
Z is a halogen atom, preferably fluorine, chlorine or bromine, a hydroxyl group, a tri-C₁-C₄-alkylsilyloxy group, or an acyl protective group which is bonded via an oxygen atom,
where the acyl protective group is an acetyl, mono-, di- or trihalogenoacetyl group, preferably with the halogen atom being fluorine or chlorine, to give a 4'-O-glycosyl-etoposide derivative of the formula I in which all the radicals R¹ to R⁸ retain their meaning as defined above, and eliminating the protective groups present in these compounds by hydrogenolysis or hydrolysis, and, where appropriate, converting by means of reductive alkylation one of the resulting compounds containing amino groups into another compound of the formula I containing dimethylamino groups.

2. The process as claimed in claim 1, wherein the acyl protective group is an acetyl, chloroacetyl or trifluoroacetyl group.

3. A 4'-O-glycosyl-etoposide of the formula I, in which
R¹ is a methyl, benzyl or 2-thienyl group,
R² is a hydrogen atom, an acyl or tri-C₁-C₄-alkylsilyl protective group,
R³ is a hydroxyl group, an acyl or tri-C₁-C₄-alkylsilyl protective group which is bonded via an oxygen atom, or an amino, acetylamino, benzyloxycarbonylamino or dimethylamino group,
R⁴ is a hydrogen atom or a methyl group,
R⁵ is a hydrogen atom, a hydroxyl group, an acyl or tri-C₁-C₄-alkylsilyl protective group which is bonded via an oxygen atom, or an amino, benzyloxycarbonylamino, azido or acetylamino group,
R⁶ is a hydroxyl group, an acyl or tri-C₁-C₄-alkylsilyl protective group which is bonded via an oxygen atom, or an amino, benzyloxycarbonylamino or azido group,
R⁷ is a hydrogen atom, an acyl or tri-C₁-C₄-alkylsilyl protective group and
R⁸ is a methyl or hydroxymethyl group or an acyl protective group which is bonded via a methyleneoxy group, or a benzyloxycarbonyl group, where the acyl protective group is an acetyl, mono-, di- or trihalogenoacetyl group with halogen being fluorine or chlorine.

4. A compound as claimed in claim 3, in which
R¹ is a methyl, benzyl or 2-thienyl group,
R² is a hydrogen atom, an acetyl or chloroacetyl group or a tri-C₁-C₄-alkylsilyl protective group,
R³ is a hydroxyl group, an acetyl, chloroacetyl or tri-C₁-C₄-alkylsilyl protective group which is bonded via an oxygen atom, or an amino, acetylamino, benzyloxycarbonylamino or dimethylamino group,
R⁴ is a hydrogen atom or a methyl group,
R⁵ is a hydrogen atom, a hydroxyl group, or an acetyl, chloroacetyl or tri-Cy-C₄-alkylsilyl protective group which is bonded via an oxygen atom, or an amino, benzyloxycarbonylamino, azido or acetylamino group,
R⁶ is a hydroxyl group, an acetyl, chloroacetyl or tri-C₁-C₄-alkylsilyl protective group which is bonded via an oxygen atom, or an amino, benzyloxycarbonylamino or azido group,
R⁷ is a hydrogen atom, an acetyl, chloroacetyl or tri-C₁-C₄-alkylsilyl protective group and
R⁸ is a methyl, hydroxymethyl, acetyloxy or chloroacetyloxymethyl group or a benzyloxycarbonyl group.

5. A pharmaceutical containing a compound as claimed in claim 3 and a functionalized tumor-specific enzyme of the formula II 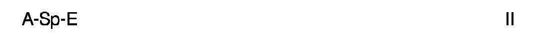 in which A is a tumor-specific antibody or a fragment thereof or a biomolecule which accumulates in a tumor, E is a glycosidase which is not immunogenic or is of low immunogenicity, and Sp (spacer) is a bifunctional sulfide-or disulfide-containing group of the formula III or IV 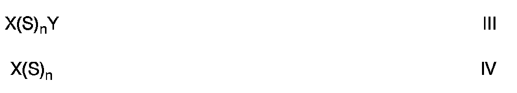 or a polypeptide spacer, in which
X or Y is -CO-R⁹⁻(N-succinimido)- or -C(=R¹⁰)-CH₂-CH₂- with R⁹ being -CH₂-CH₂-, 1,4-cyclohexylidene, 1,3-or 1,4-phenylene or methoxycarbonyl- or chloro-1,4-phenylene and R¹⁰ being O or NH, and furthermore
Y is -C (=R¹⁰) -CH₂-CH₂-, where R¹⁰ has the stated meaning, and
n is 1 or 2.

6. An agent as claimed in claim 5, wherein A-Sp-E can be prepared by genetic engineering, where A and E have the meaning as in claim 5, and Sp is an oligo- or polypeptide.

7. A functionalized tumor-specific enzyme of the formula (II) as defined in claim 5, containing a p-glucuronidase.

## Claims (Claims for the following Contracting State(s) : ES, GR)

1. A process for the preparation of a compound of the formula I 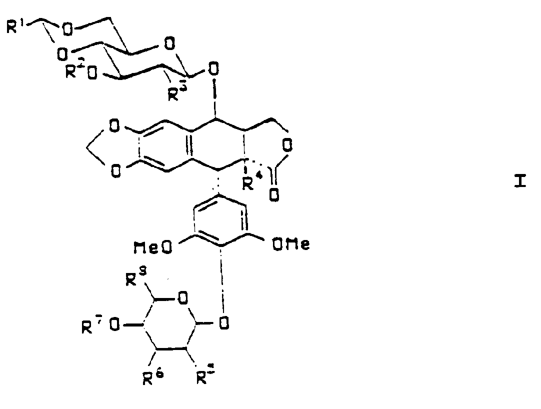 in which
R¹ is a methyl, benzyl or 2-thienyl group,
_{R}² is a hydrogen atom,
R³ is a hydroxyl, amino or dimethylamino group,
R⁴ is a hydrogen atom or a methyl group,
R⁵ is a hydrogen atom, a hydroxyl group, an amino or acetylamino group,
R⁶ is a hydroxyl group or an amino group,
R⁷ is a hydrogen atom,
R⁸ is a methyl or hydroxymethyl group or a carboxyl group or an acyl protective group which is bonded via a methyleneoxy group, or a benzyloxycarbonyl group,
where the acyl protective group is an acetyl, mono-, di- or trihalogenoacetyl group with halogen being fluorine or chlorine,
which comprises reacting, in the presence of a promoter and, where appropriate, of an acid trap or drying agent in a solvent at -50°C to 60°C, an etoposide compound of the formula V 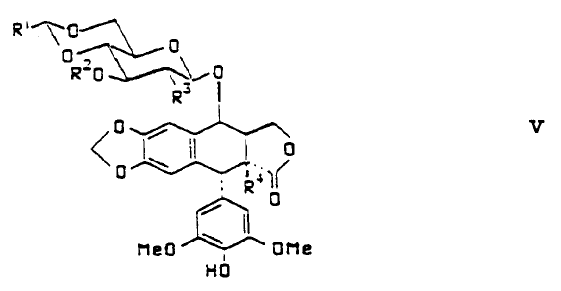 in which
R¹ is a methyl, benzyl or 2-thienyl group,
R² is a hydrogen atom, an acyl or a tri-C₁-C₄-alkylsilyl protective group,
R³ is a hydroxyl group, an acyl or tri-C₁-C₄-alkylsilyl protective group which is bonded via oxygen, or acetylamino, benzyloxycarbonylamino or dimethylamino group and
R⁴ is a hydrogen atom or a methyl group,
with a carbohydrate component of the formula VI 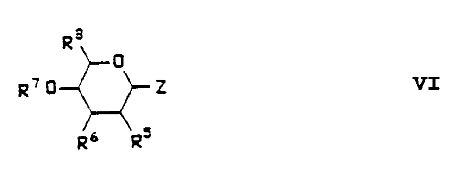 in which
R⁵ is a hydrogen atom, a hydroxyl group, an acyl protective group which is bonded via an oxygen atom, or benzyloxycarbonylamino, azido or acetylamino group,
R⁶ is an acyl protective group which is bonded via an oxygen atom, or a benzyloxycarbonylamino or azido group,
R⁷ is an acyl protective group,
R⁸ is a methyl group, methyleneoxy-acyl protective group or a benzyloxycarbonyl group and
Z is a halogen atom, preferably fluorine, chlorine or bromine, a hydroxyl group, a tri-C₁-C₄-alkylsilyloxy group, or an acyl protective group which is bonded via an oxygen atom,
where the acyl protective group is an acetyl, mono-, di- or trihalogenoacetyl group, preferably with the halogen atom being fluorine or chlorine, to give a 4'-O-glycosyl-etoposide derivative of the formula I in which all the radicals R¹ to R⁸ retain their meaning as defined above, and eliminating the protective groups present in these compounds by hydrogenolysis or hydrolysis, and, where appropriate, converting by means of reductive alkylation one of the resulting compounds containing amino groups into another compound of the formula I containing dimethylamino groups.

2. The process as claimed in claim 1, wherein the acyl protective group is an acetyl, chloroacetyl or trifluoroacetyl group.

3. The process as claimed in claim 1, wherein
R¹ is a methyl, benzyl or 2-thienyl group,
R² is a hydrogen atom, an acyl or tri-C₁-C₄-alkylsilyl protective group,
R³ is a hydroxyl group, an acyl or tri-C₁-C₄-alkylsilyl protective group, an amino, acetylamino, benzyloxycarbonylamino or dimethylamino group,
R⁴ is a hydrogen atom or a methyl group,
R⁵ is a hydrogen atom, a hydroxyl group, an acyl or tri-Ci-C4-alkylsilyl protective group which is bonded via an oxygen atom, or an amino, benzyloxycarbonylamino, azido or acetylamino group,
R⁶ is a hydroxyl group, an acyl or tri-C₁-C₄-alkylsilyl protective group which is bonded via an oxygen atom, or an amino, benzyloxycarbonylamino or azido group,
R⁷ is a hydrogen atom, an acyl or tri-C₁-C₄-alkylsilyl protective group and
R⁸ is a methyl or hydroxymethyl group or an acyl protective group which is bonded via a methyleneoxy group, or a benzyloxycarbonyl group, where the acyl protective group is an acetyl, mono-, di- or trihalogenoacetyl group with halogen being fluorine or chlorine.

4. The process as claimed in claim 1, wherein
R¹ is a methyl, benzyl or 2-thienyl group,
R² is a hydrogen atom, an acetyl or chloroacetyl group or a tri-C₁-C₄-alkylsilyl protective group,
R³ is a hydroxyl group, an acetyl, chloroacetyl or tri-C₁-C₄-alkylsilyl protective group which is bonded via an oxygen atom, or an amino, acetylamino, benzyloxycarbonylamino or dimethylamino group,
R⁴ is a hydrogen atom or a methyl group,
R⁵ is a hydrogen atom, a hydroxyl group, or an acetyl, chloroacetyl or tri-C₁-C₄-alkylsilyl protective group which is bonded via an oxygen atom, or an amino, benzyloxycarbonylamino, azido or acetylamino group,
R⁶ is a hydroxyl group, an acetyl, chloroacetyl or tri-C₁-C₄-alkylsilyl protective group which is bonded via an oxygen atom, or an amino, benzyloxycarbonylamino or azido group,
R⁷ is a hydrogen atom, an acetyl, chloroacetyl or tri-C₁-C₄-alkylsilyl protective group and
R⁸ is a methyl, hydroxymethyl, acetyloxy or chloroacetyloxymethyl group or a benzyloxycarbonyl group.

5. A process for the production of a pharmaceutical, which comprises combining a compound as claimed in claim 3 and a functionalized enzyme of the formula II 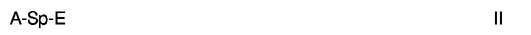 in which A is a tumor-specific antibody or a fragment thereof or a biomolecule which accumulates in a tumor, E is a glycosidase which is not immunogenic or is of low immunogenicity, and Sp (spacer) is a bifunctional sulfide-or disulfide-containing group of the formula III or IV 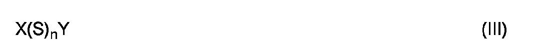 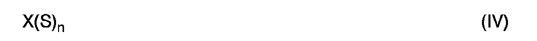 or a polypeptide spacer, in which
X or Y is -CO-R⁹⁻(N-succinimido)- or -C(=R¹⁰)-CH₂-CH₂- with R⁹ being -CH₂-cH₂-, 1,4-cyclohexylidene, 1,3- or 1,4-phenylene or methoxycarbonyl- or chloro-1,4-phenylene and R¹⁰ being O or NH, and furthermore
Y is -C(=R¹°) -CH₂-CH₂-, where R¹⁰ has the stated meaning, and
n is 1 or 2
in a suitable pharmaceutical form.

6. The process as claimed in claim 5, wherein A-Sp-E can be prepared by genetic engineering, where A and E have the meaning as in claim 5, and Sp is an oligo- or polypeptide.

7. The process as claimed in claim 5, wherein the functionalized tumor-specfic enzyme of the formula II as defined in claim 5 contains a β-glucuronidase.

## Revendications (Revendications pour l'(les) Etat(s) contractant(s) suivant(s) : AT, BE, CH, DE, DK, FR, GB, IT, LI, LU, NL, SE)

1. Procédé pour la préparation d'un composé de formule I 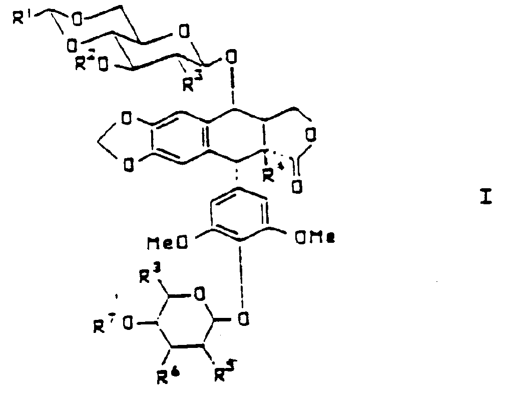 dans laquelle
R¹ est le groupe méthyle, benzyle ou 2-thiényle,
R² est un atome d'hydrogène,
R³ est le groupe hydroxyle, amino ou diméthylamino,
R⁴ est un atome d'hydrogène ou le groupe méthyle,
R⁵ est un atome d'hydrogène ou le groupe hydroxyle, amino ou acétylamino,
R⁶ est le groupe hydroxyle ou amino,
R⁷ est un atome d'hydrogène,
R⁸ est le groupe méthyle, hydroxyméthyle ou carboxyle, ou un groupe protecteur acyle relié par un groupe méthylène-oxy, ou le groupe benzyloxycarbonyle,
le groupe protecteur acyle étant un groupe acétyle, mono-, di- ou trihalogénoacétyle, dans lequel l'halogène est le fluor ou le chlore,
caractérisé en ce que l'on fait réagir un étoposide de formule v, 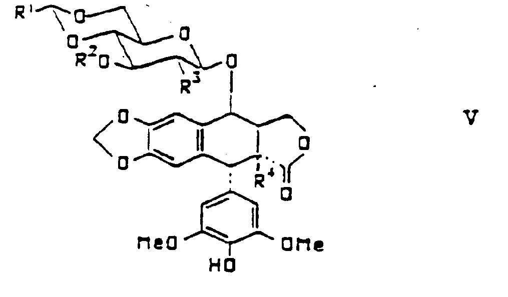 dans laquelle
R¹ représente le groupe méthyle, benzyle ou 2-thiényle,
R² représente un atome d'hydrogène, un groupe protecteur acyle ou trialkyl (Ci-C₄) silyle,
R³ représente le groupe hydroxyle, un groupe protecteur acyle relié par un atome d'oxygène ou trialkyl (C₁-C₄)-(Ci-C₄) silyle, ou le groupe acétylamino, benzyloxycarbonylamino ou diméthylamino,
R⁴ représente un atome d'hydrogène ou le groupe méthyle,
avec un composant glucidique de formule VI 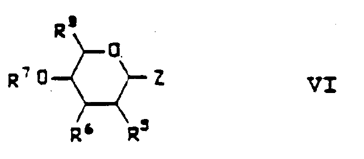 dans laquelle
R⁵ représente un atome d'hydrogène, le groupe hydroxyle, un groupe protecteur acyle relié par un atome d'oxygène, ou le groupe benzyloxycarbonylamino, azido ou acétylamino,
R⁶ représente un groupe protecteur acyle relié par un atome d'oxygène, le groupe benzyloxycarbonylamino ou azido,
R⁷ représente un groupe protecteur acyle,
R⁸ représente le groupe méthyle, un groupe protecteur acyle relié par un groupe méthylène-oxy, ou le groupe benzyloxycarbonyle, et
Z représente un atome d'halogène, de préférence de fluor, chlore ou brome, ou le groupe hydroxyle, un groupe trialkyl (Ci-C₄) silyloxy ou un groupe protecteur acyle relié par un atome d'oxygène,
le groupe protecteur acyle étant un groupe acétyle, mono-, di- ou trihalogénoacétyle, dans lequel l'halogène est de préférence le fluor ou le chlore, en présence d'un activateur et éventuellement d'un capteur d'acide ou d'un desséchant, dans un solvant, à une température de -50°C à +60°C, pour l'obtention d'un dérivé 4'-O-glycosylétoposide de formule I, dans lequel tous les radicaux R¹ à R⁸ conservent leurs définitions telles que données ci-dessus, et, dans ces composés, on élimine par hydrolyse ou hydrogénolyse les groupes protecteurs présents, et éventuellement on convertit par alkylation réductrice un des composés résultants, contenant un groupe amino, en un autre composé de formule 1 contenant un groupe diméthylamino.

2. Procédé selon la revendication 1, caractérisé en ce que le groupe protecteur acyle est le groupe acétyle, chloracétyle ou trifluoroacétyle.

3. 4'-O-glycosylétoposide de formule générale I, dans lequel
R¹ représente le groupe méthyle, benzyle ou 2-thiényle,
R² représente un atome d'hydrogène ou un groupe protecteur acyle ou trialkyl (C₁-C₄) silyle,
R³ représente le groupe hydroxyle, un groupe protecteur acyle relié par un atome d'oxygène ou trialkyl (Ci-C₄)silyle, ou le groupe amino, acétylamino, benzyloxycarbonylamino ou diméthylamino,
R⁴ représente un atome d'hydrogène ou le groupe méthyle,
R⁵ représente un atome d'hydrogène, le groupe hydroxyle, un groupe protecteur acyle relié par un atome d'oxygène ou trialkyl (C₁-C₄) silyle, ou le groupe amino, benzyloxycarbonylamino, azido ou acétylamino,
R⁶ représente le groupe hydroxyle, un groupe protecteur acyle relié par un atome d'oxygène ou trialkyl (C₁-C₄)-silyle, le groupe amino, benzyloxycarbonylamino ou azido,
R⁷ représente un atome d'hydrogène ou un groupe protecteur acyle ou trialkyl (Ci-C₄) silyle,
R⁸ représente le groupe méthyle ou hydroxyméthyle, ou un groupe protecteur acyle relié par un groupe méthy- lèneoxy, ou le groupe benzyloxycarbonyle, le groupe protecteur acyle étant le groupe acétyle ou un groupe mono-, di- ou trihalogénoacétyle, dans lequel l'halogène est le fluor ou le chlore.

4. Composé selon la revendication 3, dans lequel
R¹ représente le groupe méthyle, benzyle ou 2-thiényle,
R² représente un atome d'hydrogène ou un groupe protecteur acétyle, chloracétyle ou trialkyl (C₁-C₄) silyle,
R³ représente le groupe hydroxyle, un groupe protecteur acétyle, chloracétyle relié par un atome d'oxygène ou trialkyl (Ci-C₄) silyle, ou le groupe amino, acétylamino, benzyloxycarbonylamino ou diméthylamino,
R⁴ représente un atome d'hydrogène ou le groupe méthyle,
R⁵ représente un atome d'hydrogène, le groupe hydroxyle ou un groupe protecteur acétyle ou chloracétyle relié par un atome d'oxygène ou trialkyl (C₁-C₄) silyle, ou le groupe amino, benzyloxycarbonylamino, azido ou acétylamino,
R⁶ représente le groupe hydroxyle, un groupe protecteur acétyle ou chloracétyle relié par un atome d'oxygène ou trialkyl (C₁-C₄)-silyle, ou le groupe amino, benzyloxycarbonylamino ou azido,
R⁷ représente un atome d'hydrogène ou un groupe protecteur acétyle, chloracétyle ou trialkyl (Ci-C₄) silyle,
R⁸ représente le groupe méthyle, hydroxyméthyle, acétyloxy ou chloracétyloxyméthyle ou le groupe benzyloxycarbonyle.

5. Médicament contenant un composé selon la revendication 3 et une enzyme spécifique de tumeur, fonctionnalisée, de formule Il 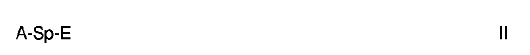 dans laquelle A représente un anticorps spécifique de tumeur ou un fragment de celui-ci, ou une biomolécule se concentrant dans une tumeur,
E représente une glycosidase non ou peu immunogène, et Sp (espaceur) représente un groupe bifonctionnel contenant un sulfure ou disulfure, de formule III ou IV 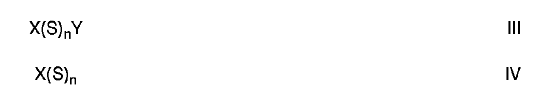 ou un espaceur polypeptidique, formules dans lesquelles
X ou Y représente un groupe -CO-R⁹-(N-succinimido)- ou -C(=R¹⁰)-CH₂-CH₂-, R⁹ étant le groupe -CH₂-CH₂-, 1,4-cyclohexylidène, 1,3- ou 1,4-phénylène ou méthoxycarbonyl- ou chloro-phén-1,4-ylène, et R¹⁰ étant O ou NH, et en outre
Y représente un groupe -C(=R¹⁰)-CH₂-CH₂-, R¹⁰ ayant la signification indiquée, et
n est 1 ou 2.

6. Médicament selon la revendication 5, caractérisé en ce que A-Sp-E peut être obtenue par génie génétique, A et E ayant les significations telles que données dans la revendication 5, et Sp représentant un oligo- ou polypeptide.

7. Enzyme spécifique de tumeur, fonctionnalisée, de formule (II) telle que définie dans la revendication 5, comprenant une β-glucuronidase.

## Revendications (Revendications pour l'(les) Etat(s) contractant(s) suivant(s) : ES, GR)

1. Procédé pour la préparation d'un composé de formule 1 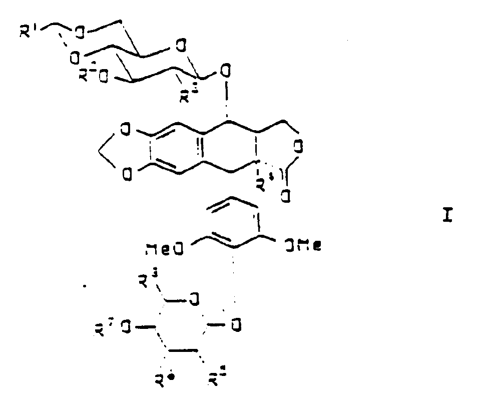 dans laquelle
R¹ est le groupe méthyle, benzyle ou 2-thiényle,
R² est un atome d'hydrogène,
R³ est le groupe hydroxyle, amino ou diméthylamino,
R⁴ est un atome d'hydrogène ou le groupe méthyle,
R⁵ est un atome d'hydrogène ou le groupe hydroxyle, amino ou acétylamino,
R⁶ est le groupe hydroxyle ou amino,
_{R}⁷ est un atome d'hydrogène,
R⁸ est le groupe méthyle, hydroxyméthyle ou carboxyle, ou un groupe protecteur acyle relié par un groupe méthylène-oxy, ou le groupe benzyloxycarbonyle,
le groupe protecteur acyle étant un groupe acétyle, mono-, di- ou trihalogénoacétyle, dans lequel l'halogène est le fluor ou le chlore,
caractérisé en ce que l'on fait réagir un étoposide de formule V; 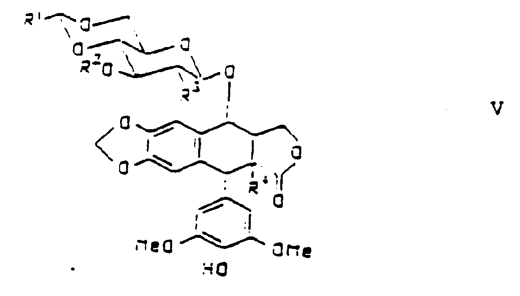 dans laquelle
R¹ représente le groupe méthyle, benzyle ou 2-thiényle,
R² représente un atome d'hydrogène, un groupe protecteur acyle ou trialkyl (C₁-C₄) silyle,
R³ représente le groupe hydroxyle, un groupe protecteur acyle relié par un atome d'oxygène ou trialkyl (C₁-C₄)-(Ci-C₄) silyle, ou le groupe acétylamino, benzyloxycarbonylamino ou diméthylamino,
R⁴ représente un atome d'hydrogène ou le groupe méthyle,
avec un composant glucidique de formule VI 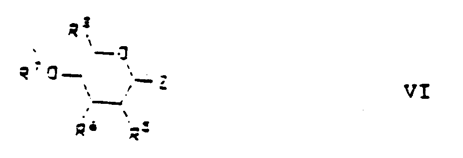 dans laquelle
R⁵ représente un atome d'hydrogène, le groupe hydroxyle, un groupe protecteur acyle relié par un atome d'oxygène, ou le groupe benzyloxycarbonylamino, azido ou acétylamino,
R⁶ représente un groupe protecteur acyle relié par un atome d'oxygène, le groupe benzyloxycarbonylamino ou azido,
R⁷ représente un groupe protecteur acyle,
R⁸ représente le groupe méthyle, un groupe protecteur acyle relié par un groupe méthylène-oxy, ou le groupe benzyloxycarbonyle, et
Z représente un atome d'halogène, de préférence de fluor, chlore ou brome, ou le groupe hydroxyle, un groupe trialkyl (C₁-C₄) silyloxy ou un groupe protecteur acyle relié par un atome d'oxygène,
le groupe protecteur acyle étant un groupe acétyle, mono-, di- ou trihalogénoacétyle, dans lequel l'halogène est de préférence le fluor ou le chlore, en présence d'un activateur et éventuellement d'un capteur d'acide ou d'un desséchant, dans un solvant, à une température de -50°C à +60°C, pour l'obtention d'un dérivé 4'-o-glycosylétoposide de formule I, dans lequel tous les radicaux R¹ à R⁸ conservent leurs définitions telles que données ci-dessus, et, dans ces composés, on élimine par hydrolyse ou hydrogénolyse les groupes protecteurs présents, et éventuellement on convertit par alkylation réductrice un des composés résultants, contenant un groupe amino, en un autre composé de formule I contenant un groupe diméthylamino.

2. Procédé selon la revendication 1, caractérisé en ce que le groupe protecteur acyle est le groupe acétyle, chloracétyle ou trifluoroacétyle.

3. Procédé selon la revendication 1, caractérisé en ce que
R¹ représente le groupe méthyle, benzyle ou 2-thiényle,
R² représente un atome d'hydrogène ou un groupe protecteur acyle ou trialkyl (C₁-C₄) silyle,
R³ représente le groupe hydroxyle, un groupe protecteur acyle ou trialkyl (C₁-C₄)-silyle, ou le groupe amino, acétylamino, benzyloxycarbonylamino ou diméthylamino,
R⁴ représente un atome d'hydrogène ou le groupe méthyle,
R⁵ représente un atome d'hydrogène, le groupe hydroxyle, un groupe protecteur acyle relié par un atome d'oxygène ou trialkyl (C₁-C₄) silyle, ou le groupe amino, benzyloxycarbonylamino, azido ou acétylamino,
R⁶ représente le groupe hydroxyle, un groupe protecteur acyle relié par un atome d'oxygène ou trialkyl (C₁-C₄)-silyle, le groupe amino, benzyloxycarbonylamino ou azido,
R⁷ représente un atome d'hydrogène ou un groupe protecteur acyle ou trialkyl (C₁-C₄) silyle,
R⁸ représente le groupe méthyle ou hydroxyméthyle, ou un groupe protecteur acyle relié par un groupe méthy- lèneoxy, ou le groupe benzyloxycarbonyle, le groupe protecteur acyle étant le groupe acétyle ou un groupe mono-, di- ou trihalogénoacétyle, dans lequel l'halogène est le fluor ou le chlore.

4. Procédé selon la revendication 3, caractérisé en ce que
R¹ représente le groupe méthyle, benzyle ou 2-thiényle,
R² représente un atome d'hydrogène ou un groupe protecteur acétyle, chloracétyle ou trialkyl (Ci-C₄) silyle,
R³ représente le groupe hydroxyle, un groupe protecteur acétyle, chloracétyle relié par un atome d'oxygène ou trialkyl (C₁-C₄) silyle, ou le groupe amino, acétylamino, benzyloxycarbonylamino ou diméthylamino,
R⁴ représente un atome d'hydrogène ou le groupe méthyle,
R⁵ représente un atome d'hydrogène, le groupe hydroxyle ou un groupe protecteur acétyle ou chloracétyle relié par un atome d'oxygène ou trialkyl (C₁-C₄) silyle, ou le groupe amino, benzyloxycarbonylamino, azido ou acétylamino,
R⁶ représente le groupe hydroxyle, un groupe protecteur acétyle ou chloracétyle relié par un atome d'oxygène ou trialkyl (C₁-C₄) silyle, ou le groupe amino, benzyloxycarbonylamino ou azido,
R⁷ représente un atome d'hydrogène ou un groupe protecteur acétyle, chloracétyle ou trialkyl (C₁-C₄) silyle,
R⁸ représente le groupe méthyle, hydroxyméthyle, acétyloxy ou chloracétyloxyméthyle ou le groupe benzyloxycarbonyle.

5. Procédé pour la fabrication d'un médicament, caractérisé en ce que l'on réunit sous une forme galénique appropriée un composé selon la revendication 3 et une enzyme fonctionnalisée de formule Il 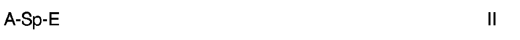 dans laquelle A représente un anticorps spécifique de tumeur ou un fragment de celui-ci, ou une biomolécule se concentrant dans une tumeur,
E représente une glycosidase non ou peu immunogène, et Sp (espaceur) représente un groupe bifonctionnel contenant un sulfure ou disulfure, de formule III ou IV 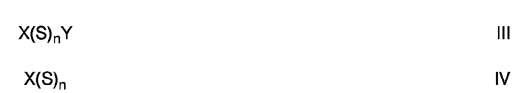 ou un espaceur polypeptidique, formules dans lesquelles
X ou Y représente un groupe -CO-R⁹-(N-succinimido)- ou -C(=R¹⁰)-CH₂-CH₂-, R⁹ étant le groupe -CH₂-CH₂-, 1,4-cyclohexylidène, 1,3-ou 1,4-phénylène ou méthoxycarbonyl- ou chloro-phén-1,4-ylène, et R¹⁰ étant O ou NH, et en outre
Y représente un groupe -C(=R¹⁰)-CH₂-CH₂-, R¹⁰ ayant la signification indiquée, et
n est 1 ou 2.

6. Procédé selon la revendication 5, caractérisé en ce que A-Sp-E peut être obtenue par génie génétique, A et E ayant les significations telles que données dans la revendication 5, et Sp représentant un oligo- ou polypeptide.

7. Procédé selon la revendication 5, caractérisé en ce que l'enzyme spécifique de tumeur, fonctionnalisée, de formule (II) telle que définie dans la revendication 5, comprend une β-glucuronidase.
